# EUROPÄISCHE PATENTSCHRIFT

(11) **EP 0 934 321 B1**
(45) Veröffentlichungstag und Bekanntmachung des Hinweises auf die Patenterteilung: **06.08.2003**
(21) Anmeldenummer: 97912139.9
(22) Anmeldetag: 08.10.1997
(51) Int. Cl.: C07D 495/04, A61K 31/505, A61K 31/38

(54) **THIENOPYRIMIDINE MIT PDE V INHIBIERENDER WIRKUNG**
THIENOPYRIMIDINE WITH PHOSPHODIESTERASE V INHIBITING EFFECT
THIENOPYRIMIDINES A EFFET INHIBITEUR DE LA PHOSPHODIESTERASE V

(30) Priorität: 24.10.1996 DE 19644228
(43) Veröffentlichungstag der Anmeldung: 11.08.1999
(73) Patentinhaber: MERCK PATENT GmbH, 64293 Darmstadt (DE)
(72) Erfinder: JONAS, Rochus, D-64291 Darmstadt (DE); SCHELLING, Pierre, D-64367 Mühltal (DE); CHRISTADLER, Maria, D-63322 Rödermark (DE); KLUXEN, Franz-Werner, D-64285 Darmstadt (DE)
(86) Internationale Anmeldenummer: EP9705530
(87) Internationale Veröffentlichungsnummer: WO98017668

(56) Entgegenhaltungen:
- EP-A- 0 201 188
- EP-A- 0 349 239
- EP-A- 0 579 496
- EP-A- 0 607 439
- EP-A- 0 640 599
- EP-A- 0 728 759
- WO-A-94/22855
- LEE,S.J. ET AL.: "Discovery of Potent Cyclic GMP Phosphodiesterase Inhibitors. 2-Pyridyl- and 2-Imidazolylquinazolines Possessing Cyclic GMP Phosphodiesterase and Thromboxane Synthesis Inhibitory Activities" J.MED.CHEM., Bd. 38, Nr. 18, 1995, WASHINGTON, Seiten 3547-3557, XP002057107
- TAKASE,Y. ET AL.: "Cyclic GMP Phosphodiesterase Inhibitors. 2. Requirement of 6-substitution of Quinazoline Derivatives for Potent and Selective Inhibitory Activity" J.MED.CHEM., Bd. 37, Nr. 13, 1994, WASHINGTON, Seiten 2106-2111, XP002056925 in der Anmeldung erwähnt
- TAKASE,Y. ET AL.: "Cyclic GMP Phosphodiesterase Inhibitors. 1. The discovery of a Novel Potent Inhibitor , 4-((3,4-(Methylenedioxy)benzyl)amino)-6,7, 8-trimethoxyquinazoline" J.MED.CHEM., Bd. 36, Nr. 24, 1993, WASHINGTON, Seiten 3765-3770, XP002056926 in der Anmeldung erwähnt

## Beschreibung

Die Erfindung betrifft Verbindungen der Formel I 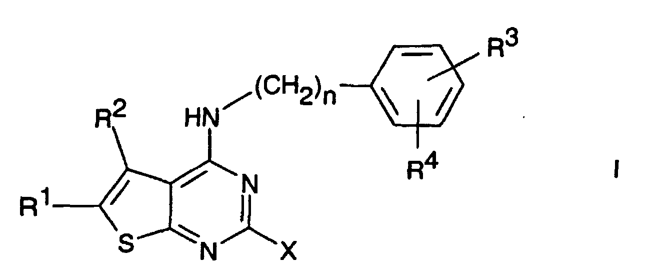 worin
- R¹, R²: jeweils unabhängig voneinander H, A, OA, Alkenyl, Alkinyl, CF₃ oder Hal,
wobei einer der Reste R¹ oder R² immer ≠ H ist,
- R¹ und R²: zusammen auch Alkylen mit 3-5 C-Atomen,
- R³, R⁴: jeweils unabhängig voneinander H, A, OA, NO₂, NH₂, NHA, NAA' oder Hal,
- R³ und R⁴: zusammen auch -O-CH₂-CH₂-, -O-CH₂-O- oder
-O-CH₂-CH₂-O-,
- X: ein- oder zweifach durch COOH, CH₂COOH, COOCH₃, COOC₂H₅, CONH₂, CON(CH₃)₂, CONHCH₃ oder CN substituiertes Phenyl, Cyclopentyl, Cyclohexyl, Cycloheptyl, 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-,-3-, -4-, -5-, -6-, -7- oder -8-isochinolyl,
- A, A': jeweils unabhängig voneinander H oder Alkyl mit 1 bis 6 C-Atomen,
- Hal: F, Cl, Br oder I und
- n: 0, 1, 2 oder 3
bedeuten,
sowie deren physiologisch unbedenklichen Salze.

Pyrimidinderivate sind beispielsweise aus der EP 201 188 oder der WO 93/06104 bekannt.

Andere heterocyclische Verbindungen als cGMP-PDE Inhibitoren sind in EP 0 728 759 A1, WO 94/22855, EP 0 607 439 A1 beschrieben. 4-Aminochinazolinderivate kennt man aus EP 0 579 496 A1, 4-Aminopyrimidinderivate als cGMP-PDE Inhibitoren sind in EP 0 640 599 A1 offenbart. Andere Pyrimidinderivate sind in EP 0 349 239 beschrieben. 2-Pyridyl- und 2-lmidazolylchinazoline sind als cGMP-PDE Inhibitoren von S. J. Lee et al. in *J. Med. Chem.* **1995,** *38*, 3547-3557 beschrieben. Y. Takase et al. beschreiben in *J. Med. Chem.* **1994,** *37*, 2106-2111 und in *J. Med. Chem.* **1993,** *36*, 3765-3770 Chinazolinderivate mit cGMP-PDE inhibitorischer Aktivität. 5-Substituierte Pyrazolo[4,3-d]pyrimidinonderivate zur Behandlung cardiovaskulärer Erkrankungen sind in EP 0 201 188 offenbart.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.
Insbesondere zeigen sie eine spezifische Inhibierung der cGMP-Phosphodiesterase (PDE V).

Chinazoline mit cGMP-Phosphodiesterase hemmender Aktivität sind z.B. in J. Med. Chem. 36, 3765 (1993) und ibid. 37, 2106 (1994) beschrieben.

Die biologische Aktivität der Verbindungen der Formel I kann nach Methoden bestimmt werden, wie sie z.B in der WO 93/06104 beschrieben sind. Die Affinität der erfindungsgemäßen Verbindungen für cGMP- und cAMP-Phosphodiesterase wird durch die Ermittlung ihrer IC₅₀-Werte (Konzentration des Inhibitors, die benötigt wird, um eine 50 %ige Inhibierung der Enzymaktivität zu erreichen) bestimmt.
Zur Durchführung der Bestimmungen können nach bekannten Methoden isolierte Enzyme verwendet werden (z.B. W.J. Thompson et al., Biochem. 1971, 10, 311). Zur Durchführung der Versuche kann eine modifizierte "batch"-Methode von W.J. Thompson und M.M. Appleman (Biochem. 1979, 18, 5228) angewendet werden.

Die Verbindungen eignen sich daher zur Behandlung von Erkrankungen des Herz-Kreislaufsystems, insbesondere der Herzinsuffizienz und zur Behandlung und/oder Therapie von Potenzstörungen (erektile Dysfunktion).

Die Verwendung von substituierten Pyrazolopyrimidinonen zur Behandlung von Impotenz ist z.B. in der WO 94/28902 beschrieben.

Die Verbindungen sind wirksam als Inhibitoren der Phenylephrin-induzierten Kontraktionen in Corpus cavemosum-Präparationen von Hasen.
Diese biologische Wirkung kann z.B. nach der Methode nachgewiesen werden, die von F. Holmquist et al. in J. Urol., 150, 1310-1315 (1993) beschrieben wird.
Die Inhibierung der Kontraktion, zeigt die Wirksamkeit der erfindungsgemäßen Verbindungen zur Therapie und/oder Behandlung von Potenzstörungen.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Verfahren zur Herstellung
a) von Verbindungen der Formel I nach Anspruch 1 sowie deren Salzen, worin X einen ein- oder zweifach durch R⁵ substituierten gesättigten 5-7-gliedrigen heterocyclischen Ring bedeutet, der über N gebunden ist,
   dadurch gekennzeichnet, daß man eine Verbindung der Formel II 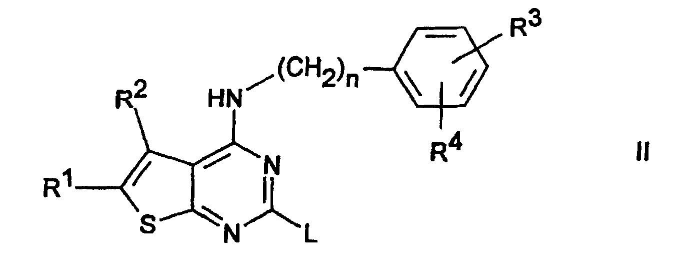 worin
   R¹, R², R³, R⁴ und n die angegebenen Bedeutungen haben,
   und L Cl, Br, OH, SCH₃ oder eine reaktionsfähige veresterte OH-Gruppe bedeutet,
   mit einem ein- oder zweifach durch R⁵ substituierten gesättigten 5-7-gliedrigen heterocyclischen Ring,
   worin R⁵ die angegebene Bedeutung hat,
   umsetzt,
   oder
b) von Verbindungen der Formel I nach Anspruch 1 sowie deren Salzen, worin X einen ein- oder zweifach durch R⁵ substituierten ungesättigten oder gesättigten 5-7-gliedrigen isocyclischen Ring bedeutet, der über C gebunden ist,
   dadurch gekennzeichnet, daß man eine Verbindung der Formel III 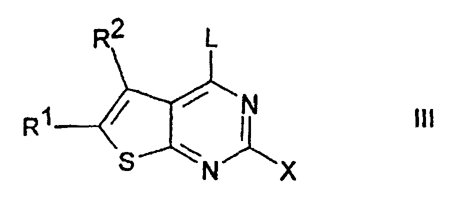 worin
   R¹, R² und X die angegebenen Bedeutungen haben,
   und L Cl, Br, OH, SCH₃ oder eine reaktionsfähige veresterte OH-Gruppe bedeutet,
   mit einer Verbindung der Formel IV 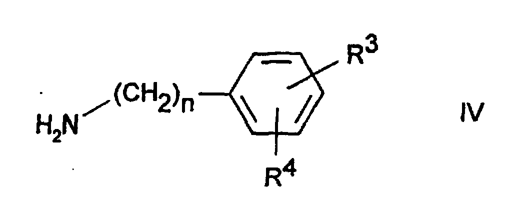 worin
   R³, R⁴ und n die angegebenen Bedeutungen haben,
   umsetzt,
   oder
c) in einer Verbindung der Formel I einen Rest R³, R⁴ und/oder X in einen anderen Rest R³, R⁴ und/oder X umwandelt, indem man einen Ester verseift oder eine Nitrogruppe reduziert,
   und/oder daß man eine saure Verbindung der Formel I durch Behandeln mit einer Base in eines ihrer Salze überführt.

Vor- und nachstehend haben die Reste R¹, R², R³, R⁴, X, L und n die bei den Formeln I, II, III, IV und V angegebenen Bedeutungen, sofern nicht ausdrücklich etwas anderes angegeben ist.

A und A' bedeuten vorzugsweise jeweils unabhängig voneinander Alkyl mit 1-6 C-Atomen.

In den vorstehenden Formeln ist Alkyl vorzugsweise unverzweigt und hat 1, 2, 3, 4, 5 oder 6 C-Atome, vorzugsweise 1, 2, 3, 4 oder 5 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, Neopentyl oder Isopentyl.

Alkylen ist vorzugsweise unverzweigt und bedeutet bevorzugt Propylen, Butylen oder Pentylen.

Von den Resten R¹ und R² steht einer vorzugsweise für H, während der andere bevorzugt Propyl oder Butyl, besonders bevorzugt aber Ethyl oder Methyl bedeutet. Ferner bedeuten R¹ und R² auch zusammen bevorzugt Propylen, Butylen oder Pentylen.

Hal bedeutet vorzugsweise F, Cl oder Br, aber auch I.

Alkenyl steht vorzugsweise für Vinyl, 1- oder 2-Propenyl, 1-Butenyl, Isobutenyl, sek.-Butenyl, ferner bevorzugt ist 1-Pentenyl, iso-Pentenyl oder 1-Hexenyl.

Alkinyl steht vorzugsweise für Ethinyl, Propin-1-yl, ferner für Butin-1-, Butin-2-yl, Pentin-1-, Pentin-2- oder Pentin-3-yl.

Die Reste R³ und R⁴ können gleich oder verschieden sein und stehen vorzugsweise in der 3- oder 4-Position des Phenylrings. Sie bedeuten beispielsweise jeweils unabhängig voneinander H, Alkyl, Alkoxy, Nitro, Amino, Alkylamino wie z.B. Methylamino, Dialkylamino wie z.B. Dimethylamino, F, Cl, Br oder I oder zusammen Ethylenoxy, Methylendioxy oder Ethylendioxy. Bevorzugt stehen sie auch jeweils für Alkoxy, wie z.B. für Methoxy, Ethoxy oder Propoxy.

Der Rest R⁵ bedeutet vorzugsweise z.B. COOH, COOCH₃, COOC₂H₅, CONH₂, CON(CH₃)₂, CONHCH₃, CN, CH₂COOH oder CH₂CH₂COOH.

Der Rest X ist vorzugsweise ein- oder zweifach durch COOH, COOCH₃, COOC₂H₅, CONH₂, CON(CH₃)₂, CONHCH₃ oder CN substituiertes Phenyl, Cyclopentyl, Cyclohexyl, Cycloheptyl, 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis le ausgedrückt werden, die der Formel I entsprechen und worin die nicht näher bezeichneten Reste die bei der Formel I angegebene Bedeutung haben, worin jedoch
in Ia
   - X: ein- oder zweifach durch COOH, COOA, CONH₂, CONAA', CONHA, CN, CH₂COOH oder CH₂CH₂COOH substituiertes Phenyl, 1-Piperidinyl oder Cyclohexyl bedeuten;
in Ib
   - R¹, R²: jeweils unabhängig voneinander H, A, OA, NO₂, CF₃ oder Hal,
   wobei mindestens einer der Reste R¹ oder R² immer ≠ H ist,
   - R³ und R⁴: zusammen -O-CH₂-CH₂-, -O-CH₂-O- oder -O-CH₂-CH₂-O,
   - X: ein- oder zweifach durch COOH, COOA, CONH₂, CONAA', CONHA, CN, CH₂COOH oder CH₂CH₂COOH substituiertes Phenyl, 1-Piperidinyl oder Cylohexyl und
   - n: 1 bedeuten;
in Ic
   - R¹, R²: jeweils unabhängig voneinander H, A, OA, NO₂, CF₃ oder Hal,
   wobei mindestens einer der Reste R¹ oder R² immer ≠ H ist,
   - R³, R⁴: jeweils unabhängig voneinander H, A, OA, Hal, NO₂, NH₂, NHA oder NAA',
   - X: ein- oder zweifach durch COOH, COOA, CONH₂, CONAA', CONHA, CN, CH₂COOH oder CH₂CH₂COOH substituiertes Phenyl, 1-Piperidinyl oder Cylohexyl und
   - n: 1 bedeuten;
in Id
   - R¹ und R²: zusammen Alkylen mit 3-5 C-Atomen,
   - R³ und R⁴: zusammen -O-CH₂-CH₂-, -O-CH₂-O- oder -O-CH₂-CH₂-O,
   - X: ein- oder zweifach durch COOH, COOA, CONH₂, CONAA', CONHA, CN, CH₂COOH oder CH₂CH₂COOH substituiertes Phenyl, 1-Piperidinyl oder Cylohexyl und
   - n: 1 bedeuten;
   - in le R¹ und R²: zusammen Alkylen mit 3-5 C-Atomen,
   - R³, R⁴: jeweils unabhängig voneinander H, A, OA, Hal, NO₂, NH₂, NHA oder NAA',
   - X: ein- oder zweifach durch COOH, COOA, CONH₂, CONAA', CONHA, CN, CH₂COOH oder CH₂CH₂COOH substituiertes Phenyl, 1-Piperidinyl oder Cylohexyl und
   - n: 1 bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II, III und IV haben R¹, R², R³, R⁴, X und n die angegebenen Bedeutungen, insbesondere die angegebenen bevorzugten Bedeutungen.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyloder p-Tolylsulfonyloxy, ferner auch 2-Naphthalinsulfonyloxy).

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Verbindungen der Formel I, worin X über N an das Thienopyrimidin-Ringsystem gebunden ist, können vorzugsweise erhalten werden, indem man Verbindungen der Formel II mit einem unsubstituierten oder ein- oder zweifach durch COOH, COOA, CONH₂, CONAA', CONHA oder CN substituierten gesättigten 5-7-gliedrigen heterocyclischen Ring umsetzt.

Die Ausgangsstoffe der Formeln II sind teilweise bekannt. Sofern sie nicht bekannt sind, können sie nach an sich bekannten Methoden hergestellt werden.

Vorstufen der Verbindungen der Formel II können z.B. durch Cyclisierung und Halogenierung analog J. Med. Chem. 24, 374 (1981) hergestellt werden. Durch anschließende Umsetzung mit Arylalkylaminen erhält man die Verbindungen der Formel II.

Im einzelnen erfolgt die Umsetzung der Verbindungen der Formel II mit dem NH-haltigen Heterocyclus in Gegenwart oder Abwesenheit eines inerten Lösungsmittels bei Temperaturen zwischen etwa -20 und etwa 150°, vorzugsweise zwischen 20 und 100°.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Verbindungen der Formel I, worin X über C an das Thienopyrimidin-Ringsystem gebunden ist, können weiterhin erhalten werden, indem man Verbindungen der Formel III mit Verbindungen der Formel IV umsetzt.
Die Ausgangsverbindungen der Formel IV und V sind in der Regel bekannt. Sind sie nicht bekannt, so können sie nach an sich bekannten Methoden hergestellt werden.
Verbindungen der Formel III können z.B. durch Umsetzung mit POCl₃ aus Verbindungen erhalten werden, die aus Thiophenderivaten und CNsubstituierten Isocyclen aufgebaut werden (Eur. J. Med. Chem. 23, 453 (1988).

Die Umsetzung der Verbindungen der Formel III mit Verbindungen der Formel IV erfolgt unter ähnlichen Bedingungen, betreffend die Reaktionszeit, Temperatur und Lösungsmittel, wie dies für die Umsetzung der Verbindungen der Formel II mit den NH-haltigen Heterocyclen beschrieben ist.

Es ist ferner möglich, in einer Verbindung der Formel I einen Rest R³ und/oder R⁴ in einen anderen Rest R³ und/oder R⁴ umzuwandeln, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert oder Cyangruppen zu COOH-Gruppen hydrolysiert.

Eine Säure der Formel I kann mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann die Säure der Formel I mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.

Andererseits kann eine Base der Formel I mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind auch Arzneimittel der Formel I und ihre physiologisch unbedenklichen Salze als Phosphodiesterase V-Hemmer.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cGMP(cydo-Guanosin-monophosphat)-Spiegels zu Entzündungshemmung oder -verhinderung und Muskelentspannung führt, eingesetzt werden. Besondere Verwendung können die erfindungsgemäßen Verbindungen bei der Behandlung von Krankheiten des Herz-Kreislaufsystems und zur Behandlung und/oder Therapie von Potenzstörungen finden.

Dabei werden die Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.
Massenspektrometrie (MS): EI (Elektronenstoß-lonisation) M⁺
FAB (Fast Atom Bombardment) (M+H)⁺

### Beispiel 1

Eine Lösung von 3,29 g 2,4-Dichlor-6-methyl-thieno-[2,3-d]-pyrimidin in 80 ml Dichlormethan wird mit 3,02 g 3,4-Methylendioxybenzylamin ("A") versetzt und nach Zugabe von 1,52 g Triethylamin 12 Stunden bei Raumtemperatur gerührt. Das Lösungsmittel wird entfernt und wie üblich aufgearbeitet. Man erhält 3,38 g 2-Chlor-6-methyl-4-(3,4-methylendioxybenzylamino)-thieno-[2,3-d]-pyrimidin, F. 162°.

Analog erhält man durch Umsetzung von "A"
mit 2,4-Dichlor-5-methyl-thieno-[2,3-d]-pyrimidin
   2-Chlor-5-methyl-4-(3,4-methylendioxybenzylamino)-thieno-[2,3-d]-pyrimidin;
mit 2,4-Dichlor-5,6,7,8-tetrahydro-[1]-benzothieno-[2,3-d]-pyrimidin
   2-Chlor-5,6,7,8-tetrahydro-4-(3,4-methylendioxybenzylamino)-[1]-benzothieno-[2,3-d]-pyrimidin, F. 222°;
mit 2,4-Dichlor-5,6-cyclopenteno-[1]-benzothieno-[2,3-d]-pyrimidin
   2-Chlor-5,6-cyclopenteno-4-(3,4-methylendioxybenzylamino)-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-(3,4-methylendioxybenzylamino)-[1]-benzothieno-[2,3-d]-pyrimidin;
mit 2,4-Dichlor-6-ethyl-thieno-[2,3-d]-pyrimidin
   2-Chlor-6-ethyl-4-(3,4-methylendioxybenzylamino)-thieno-[2,3-d]-pyrimidin, F. 148°;
mit 2,4,6-Trichlor-thieno-[2,3-d]-pyrimidin
   2,6-Dichlor-4-(3,4-methylendioxybenzylamino)-thieno-[2,3-d]-pyrimidin;
mit 2,4,5-Trichlor-6-methyl-thieno-[2,3-d]-pyrimidin
   2,5-Dichlor-6-methyl-4-(3,4-methylendioxybenzylamino)-thieno-[2,3-d]-pyrimidin;
mit 2,4-Dichlor-6-nitro-thieno-[2,3-d]-pyrimidin
   2-Chlor-6-nitro-4-(3,4-methylendioxybenzylamino)-thieno-[2,3-d]-pyrimidin;
mit 2,4-Dichlor-5,6-dimethyl-thieno-[2,3-d]-pyrimidin
   2-Chlor-5,6-dimethyl-4-(3,4-methylendioxybenzylamino)-thieno-[2,3-d]-pyrimidin;
mit 2,4-Dichlor-6-trifluormethyl-thieno-[2,3-d]-pyrimidin
   2-Chlor-6-trifluormethyl-4-(3,4-methylendioxybenzylamino)-thieno-[2,3-d]-pyrimidin.

Analog erhält man durch Umsetzung von 3-Chlor-4-methoxy-benzylamin
mit 2,4-Dichlor-6-methyl-thieno-[2,3-d]-pyrimidin
   2-Chlor-6-methyl-4-(3-chlor-4-methoxybenzylamino)-thieno-[2,3-d]-pyrimidin;
mit 2,4-Dichlor-5-methyl-thieno-[2,3-d]-pyrimidin
   2-Chlor-5-methyl-4-(3-chlor-4-methoxybenzylamino)-thieno-[2,3-d]-pyrimidin;
mit 2,4-Dichlor-5,6,7,8-tetrahydro-[1]-benzothieno-[2,3-d]-pyrimidin
   2-Chlor-5,6,7,8-tetrahydro-4-(3-chlor-4-methoxybenzylamino)-[1]-[1]-benzothieno-[2,3-d]-pyrimidin;
mit 2,4-Dichlor-5,6-cyclopenteno-[1]-benzothieno-[2,3-d]-pyrimidin
   2-Chlor-5,6-cyclopenteno-4-(3-chlor-4-methoxybenzylamino)-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-(3-chlor-4-methoxybenzylamino)-[1]-benzothieno-[2,3-d]-pyrimidin;
mit 2,4-Dichlor-6-ethyl-thieno-[2,3-d]-pyrimidin
   2-Chlor-6-ethyl-4-(3-chlor-4-methoxybenzylamino)-thieno-[2,3-d]-pyrimidin;
mit 2,4,6-Trichlor-thieno-[2,3-d]-pyrimidin
   2,6-Dichlor-4-(3-chlor-4-methoxybenzylamino)-thieno-[2,3-d]-pyrimidin;
mit 2,4,5-Trichlor-6-methyl-thieno-[2,3-d]-pyrimidin
   2,5-Dichlor-6-methyl-4-(3-chlor-4-methoxybenzylamino)-thieno-[2,3-d]-pyrimidin;
mit 2,4-Dichlor-6-nitro-thieno-[2,3-d]-pyrimidin
   2-Chlor-6-nitro-4-(3-chlor-4-methoxybenzylamino)-thieno-[2,3-d]-pyrimidin;
mit 2,4-Dichlor-5,6-dimethyl-thieno-[2,3-d]-pyrimidin
   2-Chlor-5,6-dimethyl-4-(3-chlor-4-methoxybenzylamino)-thieno-[2,3-d]-pyrimidin;
mit 2,4-Dichlor-6-trifluormethyl-thieno-[2,3-d]-pyrimidin
   2-Chlor-6-trifluormethyl-4-(3-chlor-4-methoxybenzylamino)-thieno-[2,3-d]-pyrimidin.

Analog erhält man durch Umsetzung von 3,4-Dimethoxybenzylamin
mit 2,4-Dichlor-6-methyl-thieno-[2,3-d]-pyrimidin
   2-Chlor-6-methyl-4-(3,4-dimethoxybenzylamino)-thieno-[2,3-d]-pyrimidin;
mit 2,4-Dichlor-5-methyl-thieno-[2,3-d]-pyrimidin
   2-Chlor-5-methyl-4-(3,4-dimethoxybenzylamino)-thieno-[2,3-d]-pyrimidin;
mit 2,4-Dichlor-5,6,7,8-tetrahydro-[1]-[1]-benzothieno-[2,3-d]-pyrimidin
   2-Chlor-5,6,7,8-tetrahydro-4-(3,4-dimethoxybenzylamino)-[1]-[1]-benzothieno-[2,3-d]-pyrimidin;
mit 2,4-Dichlor-5,6-cyclopenteno-[1]-benzothieno-[2,3-d]-pyrimidin
   2-Chlor-5,6-cyclopenteno-4-(3,4-dimethoxybenzylamino)-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-(3,4-dimethoxybenzylamino)-[1]-benzothieno-[2,3-d]-pyrimidin;
mit 2,4-Dichlor-6-ethyl-thieno-[2,3-d]-pyrimidin
   2-Chlor-6-ethyl-4-(3,4-dimethoxybenzylamino)-thieno-[2,3-d]-pyrimidin;
mit 2,4,6-Trichlor-thieno-[2,3-d]-pyrimidin
   2,6-Dichlor-4-(3,4-dimethoxybenzylamino)-thieno-[2,3-d]-pyrimidin;
mit 2,4,5-Trichlor-6-methyl-thieno-[2,3-d]-pyrimidin
   2,5-Dichlor-6-methyl-4-(3,4-dimethoxybenzylamino)-thieno-[2,3-d]-pyrimidin;
mit 2,4-Dichlor-6-nitro-thieno-[2,3-d]-pyrimidin
   2-Chlor-6-nitro-4-(3,4-dimethoxybenzylamino)-thieno-[2,3-d]-pyrimidin;
mit 2,4-Dichlor-5,6-dimethyl-thieno-[2,3-d]-pyrimidin
   2-Chlor-5,6-dimethyl-4-[3,4-dimethoxybenzylamino)-thieno-[2,3-d]-pyrimidin;
mit 2,4-Dichlor-6-trifluormethyl-thieno-[2,3-d]-pyrimidin
   2-Chlor-6-trifluormethyl-4-(3,4-dimethoxybenzylamino)-thieno-[2,3-d]-pyrimidin.

Analog erhält man durch Umsetzung von Benzylamin
mit 2,4-Dichlor-6-methyl-thieno-[2,3-d]-pyrimidin
   2-Chlor-6-methyl-4-benzylamino-thieno-[2,3-d]-pyrimidin;
mit 2,4-Dichlor-5-methyl-thieno-[2,3-d]-pyrimidin
   2-Chlor-5-methyl-4-benzylamino-thieno-[2,3-d]-pyrimidin;
mit 2,4-Dichlor-5,6,7,8-tetrahydro-[1]-[1]-benzothieno-[2,3-d]-pyrimidin
   2-Chlor-5,6,7,8-tetrahydro-4-benzylamino-[1]-[1]-benzothieno-[2,3-d]-pyrimidin;
mit 2,4-Dichlor-5,6-cyclopenteno-[1]-benzothieno-[2,3-d]-pyrimidin
   2-Chlor-5,6-cyclopenteno-4-benzylamino-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-benzylamino-[1]-benzothieno-[2,3-d]-pyrimidin;
mit 2,4-Dichlor-6-ethyl-thieno-[2,3-d]-pyrimidin
   2-Chlor-6-ethyl-4-benzylamino-thieno-[2,3-d]-pyrimidin;
mit 2,4,6-Trichlor-thieno-[2,3-d]-pyrimidin
   2,6-Dichlor-4-benzylamino-thieno-[2,3-d]-pyrimidin;
mit 2,4,5-Trichlor-6-methyl-thieno-[2,3-d]-pyrimidin
   2,5-Dichlor-6-methyl-4-benzylamino-thieno-[2,3-d]-pyrimidin;
mit 2,4-Dichlor-6-nitro-thieno-[2,3-d]-pyrimidin
   2-Chlor-6-nitro-4-benzylamino-thieno-[2,3-d]-pyrimidin;
mit 2,4-Dichlor-5,6-dimethyl-thieno-[2,3-d]-pyrimidin
   2-Chlor-5,6-dimethyl-4-benzylamino-thieno-[2,3-d]-pyrimidin;
mit 2,4-Dichlor-6-trifluormethyl-thieno-[2,3-d]-pyrimidin
   2-Chlor-6-trifluormethyl-4-benzylamino-thieno-[2,3-d]-pyrimidin.

Analog erhält man durch Umsetzung von 4-Fluorbenzylamin
mit 2,4-Dichlor-6-methyl-thieno-[2,3-d]-pyrimidin
   2-Chlor-6-methyl-4-(4-fluorbenzylamino)-thieno-[2,3-d]-pyrimidin;
mit 2,4-Dichlor-5-methyl-thieno-[2,3-d]-pyrimidin
   2-Chlor-5-methyl-4-(4-fluorbenzylamino)-thieno-[2,3-d]-pyrimidin;
mit 2,4-Dichlor-5,6,7,8-tetrahydro-[1]-benzothieno-[2,3-d]-pyrimidin
   2-Chlor-5,6,7,8-tetrahydro-4-(4-fluorbenzylamino)-[1]-benzothieno-[2,3-d]-pyrimidin;
mit 2,4-Dichlor-5,6-cyclopenteno-[1]-benzothieno-[2,3-d]-pyrimidin
   2-Chlor-5,6-cyclopenteno-4-(4-fluorbenzylamino)-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-(4-fluorbenzylamino)-[1]-benzothieno-[2,3-d]-pyrimidin;
mit 2,4-Dichlor-6-ethyl-thieno-[2,3-d]-pyrimidin
   2-Chlor-6-ethyl-4-(4-fluorbenzylamino)-thieno-[2,3-d]-pyrimidin;
mit 2,4,6-Trichlor-thieno-[2,3-d]-pyrimidin
   2,6-Dichlor-4-(4-fluorbenzylamino)-thieno-[2,3-d]-pyrimidin;
mit 2,4,5-Trichlor-6-methyl-thieno-[2,3-d]-pyrimidin
   2,5-Dichlor-6-methyl-4-(4-fluorbenzylamino)-thieno-[2,3-d]-pyrimidin;
mit 2,4-Dichlor-6-nitro-thieno-[2,3-d]-pyrimidin
   2-Chlor-6-nitro-4-(4-fluorbenzylamino)-thieno-[2,3-d]-pyrimidin;
mit 2,4-Dichlor-5,6-dimethyl-thieno-[2,3-d]-pyrimidin
   2-Chlor-5,6-dimethyl-4-(4-fluorbenzylamino)-thieno-[2,3-d]-pyrimidin;
mit 2,4-Dichlor-6-trifluormethyl-thieno-[2,3-d]-pyrimidin
   2-Chlor-6-trifluormethyl-4-(4-fluorbenzylamino)-thieno-[2,3-d]-pyrimidin.

Analog erhält man durch Umsetzung von 3,4-Dichlorbenzylamin
mit 2,4-Dichlor-6-methyl-thieno-[2,3-d]-pyrimidin
   2-Chlor-6-methyl-4-(3,4-dichlorbenzylamino)-thieno-[2,3-d]-pyrimidin;
mit 2,4-Dichlor-5-methyl-thieno-[2,3-d]-pyrimidin
   2-Chlor-5-methyl-4-(3,4-dichlorbenzylamino)-thieno-[2,34]-pyrimidin;
mit 2,4-Dichlor-5,6,7,8-tetrahydro-[1]-benzothieno-[2,3-d]-pyrimidin
   2-Chlor-5,6,7,8-tetrahydro-4-(3,4-dichlorbenzylamino)-[1]-benzothieno-[2,3-d]-pyrimidin;
mit 2,4-Dichlor-5,6-cyclopenteno-[1]-benzothieno-[2,3-d]-pyrimidin
   2-Chlor-5,6-cyclopenteno-4-(3,4-dichlorbenzylamino)-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-(3,4-dichlorbenzylamino)-[1]-benzothieno-[2,3-d]-pyrimidin;
mit 2,4-Dichlor-6-ethyl-thieno-[2,3-d]-pyrimidin
   2-Chlor-6-ethyl-4-(3,4-dichlorbenzylamino)-thieno-[2,3-d]-pyrimidin;
mit 2,4,6-Trichlor-thieno-[2,3-d]-pyrimidin
   2,6-Dichlor-4-(3,4-dichlorbenzylamino)-thieno-[2,3-d]-pyrimidin;
mit 2,4,5-Trichlor-6-methyl-thieno-[2,3-d]-pyrimidin
   2,5-Dichlor-6-methyl-4-(3,4-dichlorbenzylamino)-thieno-[2,3-d]-pyrimidin;
mit 2,4-Dichlor-6-nitro-thieno-[2,3-d]-pyrimidin
   2-Chlor-6-nitro-4-(3,4-dichlorbenzylamino)-thieno-[2,3-d]-pyrimidin;
mit 2,4-Dichlor-5,6-dimethyl-thieno-[2,3-d]-pyrimidin
   2-Chlor-5,6-dimethyl-4-(3,4-dichlorbenzylamino)-thieno-[2,3-d]-pyrimidin;
mit 2,4-Dichlor-6-trifluormethyl-thieno-[2,3-d]-pyrimidin
   2-Chlor-6-trifluormethyl-4-(3,4-dichlorbenzylamino)-thieno-[2,3-d]-pyrimidin.

Analog erhält man durch Umsetzung von 3-Nitrobenzylamin
mit 2,4-Dichlor-6-methyl-thieno-[2,3-d]-pyrimidin
   2-Chlor-6-methyl-4-(3-nitrobenzylamino)-thieno-[2,3-d]-pyrimidin;
mit 2,4-Dichlor-5-methyl-thieno-[2,3-d]-pyrimidin
   2-Chlor-5-methyl-4-(3-nitrobenzylamino)-thieno-[2,3-d]-pyrimidin;
mit 2,4-Dichlor-5,6,7,8-tetrahydro-[1]-benzothieno-[2,3-d]-pyrimidin
   2-Chlor-5,6,7,8-tetrahydro-4-(3-nitrobenzylamino)-[1]-benzothieno-[2,3-d]-pyrimidin;
mit 2,4-Dichlor-5,6-cyclopenteno-[1]-benzothieno-[2,3-d]-pyrimidin
   2-Chlor-5,6-cyclopenteno-4-(3-nitrobenzylamino)-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-(3-nitrobenzylamino)-[1]-benzothieno-[2,3-d]-pyrimidin;
mit 2,4-Dichlor-6-ethyl-thieno-[2,3-d]-pyrimidin
   2-Chlor-6-ethyl-4-(3-nitrobenzylamino)-thieno-[2,3-d]-pyrimidin;
mit 2,4,6-Trichlor-thieno-[2,3-d]-pyrimidin
   2,6-Dichlor-4-(3-nitrobenzylamin)-thieno-[2,3-d]-pyrimidin;
mit 2,4,5-Trichlor-6-methyl-thieno-[2,3-d]-pyrimidin
   2,5-Dichlor-6-methyl-4-(3-nitrobenzylamino)-thieno-[2,3-d]-pyrimidin;
mit 2,4-Dichlor-6-nitro-thieno-[2,3-d]-pyrimidin
   2-Chlor-6-nitro-4-(3-nitrobenzylamino)-thieno-[2,3-d]-pyrimidin;
mit 2,4-Dichlor-5,6-dimethyl-thieno-[2,3-d]-pyrimidin
   2-Chlor-5,6-dimethyl-4-(3-nitrobenzylamino)-thieno-[2,3-d]-pyrimidin;
mit 2,4-Dichlor-6-trifluormethyl-thieno-[2,3-d]-pyrimidin
   2-Chlor-6-trifluormethyl-4-(3-nitrobenzylamino)-thieno-[2,3-d]-pyrimidin.

Analog erhält man durch Umsetzung von 3,4-Methylendioxyphenethylamin
mit 2,4-Dichlor-6-methyl-thieno-[2,3-d]-pyrimidin
   2-Chlor-6-methyl-4-(3,4-methylendioxyphenethylamino)-thieno-[2,3-d]-pyrimidin;
mit 2,4-Dichlor-5-methyl-thieno-[2,3-d]-pyrimidin
   2-Chlor-5-methyl-4-(3,4-methylendioxyphenethylamino)-thieno-[2,3-d]-pyrimidin;
mit 2,4-Dichlor-5,6,7,8-tetrahydro-[1]-benzothieno-[2,3-d]-pyrimidin
   2-Chlor-5,6,7,8-tetrahydro-4-(3,4-methylendioxyphenethylamino)-[1]-benzothieno-[2,3-d]-pyrimidin;
mit 2,4-Dichlor-5,6-cyclopenteno-[1]-benzothieno-[2,3-d]-pyrimidin
   2-Chlor-5,6-cyclopenteno-4-(3,4-methylendioxyphenethylamino)-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-(3,4-methylendioxyphenethylamino)-[1]-benzothieno-[2,3-d]-pyrimidin;
mit 2,4-Dichlor-6-ethyl-thieno-[2,3-d]-pyrimidin
   2-Chlor-6-ethyl-4-(3,4-methylendioxyphenethylamino)-thieno-[2,3-d]-pyrimidin;
mit 2,4,6-Trichlor-thieno-[2,3-d]-pyrimidin
   2,6-Dichlor-4-(3,4-methylendioxyphenethylamino)-thieno-[2,3-d]-pyrimidin;
mit 2,4,5-Trichlor-6-methyl-thieno-[2,3-d]-pyrimidin
   2,5-Dichlor-6-methyl-4-(3,4-methylendioxyphenethylamino)-thieno-[2,3-d]-pyrimidin;
mit 2,4-Dichlor-6-nitro-thieno-[2,3-d]-pyrimidin
   2-Chlor-6-nitro-4-(3,4-methylendioxyphenethylamino)-thieno-[2,3-d]-pyrimidin;
mit 2,4-Dichlor-5,6-dimethyl-thieno-[2,3-d]-pyrimidin
   2-Chlor-5,6-dimethyl-4-(3,4-methylendioxyphenethylamino)-thieno-[2,3-d]-pyrimidin;
mit 2,4-Dichlor-6-trifluormethyl-thieno-[2,3-d]-pyrimidin
   2-Chlor-6-trifluormethyl-4-(3,4-methylendioxyphenethylamino)-thieno-[2,3-d]-pyrimidin.

Analog erhält man durch Umsetzung von 3,4-Ethylendioxybenzylamin
mit 2,4-Dichlor-6-methyl-thieno-[2,3-d]-pyrimidin
   2-Chlor-6-methyl-4-(3,4-ethylendioxybenzylamino)-thieno-[2,3-d]-pyrimidin;
mit 2,4-Dichlor-5-methyl-thieno-[2,3-d]-pyrimidin
   2-Chlor-5-methyl-4-(3,4-ethylendioxybenzylamino)-thieno-[2,3-d]-pyrimidin;
mit 2,4-Dichlor-5,6,7,8-tetrahydro-[1]-benzothieno-[2,3-d]-pyrimidin
   2-Chlor-5,6,7,8-tetrahydro-4-(3,4-ethylendioxybenzylamino)-[1]-benzothieno-[2,3-d]-pyrimidin;
mit 2,4-Dichlor-5,6-cyclopenteno-[1]-benzothieno-[2,3-d]-pyrimidin
   2-Chlor-5,6-cyclopenteno-4-(3,4-ethylendioxybenzylamino)-[1]-benzothieno-[2,3-d]-pyrimidin;
mit 2,4-Dichlor-5,6-cyclohepteno-[1]-benzothieno-[2,3-d]-pyrimidin
   2-Chlor-5,6-cyclohepteno-4-(3,4-ethylendioxybenzylamino)-[1]-benzothieno-[2,3-d]-pyrimidin;
mit 2,4-Dichlor-6-ethyl-thieno-[2,3-d]-pyrimidin
   2-Chlor-6-ethyl-4-(3,4-ethylendioxybenzylamino)-thieno-[2,3-d]-pyrimidin;
mit 2,4,6-Trichlor-thieno-[2,3-d]-pyrimidin
   2,6-Dichlor-4-(3,4-ethylendioxybenzylamino)-thieno-[2,3-d]-pyrimidin;
mit 2,4,5-Trichlor-6-methyl-thieno-[2,3-d]-pyrimidin
   2,5-Dichlor-6-methyl-4-(3,4-ethylendioxybenzylamino)-thieno-[2,3-d]-pyrimidin;
mit 2,4-Dichlor-6-nitro-thieno-[2,3-d]-pyrimidin
   2-Chlor-6-nitro-4-(3,4-ethylendioxybenzylamino)-thieno-[2,3-d]-pyrimidin;
mit 2,4-Dichlor-5,6-dimethyl-thieno-[2,3-d]-pyrimidin
   2-Chlor-5,6-dimethyl-4-(3,4-ethylendioxybenzylamino)-thieno-[2,3-d]-pyrimidin;
mit 2,4-Dichlor-6-trifluormethyl-thieno-[2,3-d]-pyrimidin
   2-Chlor-6-trifluormethyl-4-(3,4-ethylendioxybenzylamino)-thieno-[2,3-d]-pyrimidin.

### Beispiel 2

1,67 g 2-Chlor-6-methyl-4-(3,4-methylendioxybenzylamino)-thieno-[2,3-d]-pyrimidin und 3 g Piperidin-4-carbonsäureethylester werden 3 Stunden bei 130 ° erhitzt. Nach Abkühlen wird der Rückstand in Dichlormethan gelöst und wie üblich aufgearbeitet. Man erhält 0,5 g 1-[6-Methyl-4-(3,4-methylendioxybenzylamino)-thieno-[2,3-d]-pyrimidin-2-yl]-piperidin-4-carbonsäureethylester.

Analog erhält man durch Umsetzung von Piperidin-4-carbonsäureethylester mit den unter Beispiel 1 erhaltenen 2-Chlor-thieno-[2,3-d]-pyrimidin-Derivaten, die in 4-Stellung Arylalkylamino-substituiert sind, die nachstehenden Verbindungen
1-[5-Methyl-4-(3,4-methylendioxybenzylamino)-thieno-[2,3-d]-pyrimidin-2-yl]-piperidin-4-carbonsäureethylester;
1-[5,6,7,8-Tetrahydro-4-(3,4-methylendioxybenzylamino)-[1]-benzothieno-[2,3-d]-pyrimidin-2-yl]-piperidin-4-carbonsäureethylester;
1-[5,6-Cyclopenteno-4-(3,4-methylendioxybenzylamino)-[1]-benzothieno-[2,3-d]-pyrimidin-2-yl]-piperidin-4-carbonsäureethylester;
1-[5,6-Cyclohepteno-4-(3,4-methylendioxybenzylamino)-[1]-benzothieno-[2,3-d]-pyrimidin-2-yl]-piperidin-4-carbonsäureethylester;
1-[6-Ethyl-4-(3,4-methylendioxybenzylamino)-thieno-[2,3-d]-pyrimidin-2-yl]-piperidin-4-carbonsäureethylester
1-[6-Chlor-4-(3,4-methylendioxybenzylamino)-thieno-[2,3-d]-pyrimidin-2-yl]-piperidin-4-carbonsäureethylester;
1-[5-Chlor-6-methyl-4-(3,4-methylendioxybenzylamino)-thieno-[2,3-d]-pyrimidin-2-yl]-piperidin-4-carbonsäureethylester;
1-[6-Nitro-4-(3,4-methylendioxybenzylamino)-thieno-[2,3-d]-pyrimidin-2-yl]-piperidin-4-carbonsäureethylester;
1-[5,6-Dimethyl-4-(3,4-methylendioxybenzylamino)-thieno-[2,3-d]-pyrimidin-2-yl]-piperidin-4-carbonsäureethylester;
1-[6-Trifluormethyl-4-(3,4-methylendioxybenzylamino)-thieno-[2,3-d]-pyrimidin-2-yl]-piperidin-4-carbonsäureethylester;
1-[6-Methyl-4-(3-chlor-4-methoxybenzylamino)-thieno-[2,3-d]-pyrimidin-2-yl]-piperidin-4-carbonsäureethylester;
1-[5-Methyl-4-(3-chlor-4-methoxybenzylamino)-thieno-[2,3-d]-pyrimidin-2-yl]-piperidin-4-carbonsäureethylester;
1-[5,6,7,8-Tetrahydro-4-(3-chlor-4-methoxybenzylamino)-[1]-benzothieno-[2,3-d]-pyrimidin-2-yl]-piperidin-4-carbonsäureethylester;
1-[5,6-Cyclopenteno-4-(3-chlor-4-methoxybenzylamino)-[1]-benzothieno-[2,3-d]-pyrimidin-2-yl]-piperidin-4-carbonsäureethylester;
1-[5,6-Cyclohepteno-4-(3-chlor-4-methoxybenzylamino)-[1]-benzothieno-[2,3-d]-pyrimidin-2-yl]-piperidin-4-carbonsäureethylester;
1-[6-Ethyl-4-(3-chlor-4-methoxybenzylamino)-thieno-[2,3-d]-pyrimidin-2-yl]-piperidin-4-carbonsäureethylester;
1-[6-Chlor-4-(3-chlor-4-methoxybenzylamino)-thieno-[2,3-d]-pyrimidin-2-yl]-piperidin-4-carbonsäureethylester;
1-[5-Chlor-6-methyl-4-(3-chlor-4-methoxybenzylamino)-thieno-[2,3-d]-pyrimidin-2-yl]-piperidin-4-carbonsäureethylester;
1-[6-Nitro-4-(3-chlor-4-methoxybenzylamino)-thieno-[2,3-d]-pyrimidin-2-yl]-piperidin-4-carbonsäureethylester;
1-[5,6-Dimethyl-4-(3-chlor-4-methoxybenzylamino)-thieno-[2,3-d]-pyrimidin-2-yl]-piperidin-4-carbonsäureethylester;
1-[6-Trifluormethyl-4-(3-chlor-4-methoxybenzylamino)-thieno-[2,3-d]-pyrimidin-2-yl]-piperidin-4-carbonsäureethylester;
1-[6-Methyl-4-(3,4-dimethoxybenzylamino)-thieno-[2,3-d]-pyrimidin-2-yl]-piperidin-4-carbonsäureethylester;
1-[5-Methyl-4-(3,4-dimethoxybenzylamino)-thieno-[2,3-d]-pyrimidin-2-yl]-piperidin-4-carbonsäureethylester;
1-[5,6,7,8-Tetrahydro-4-(3,4-dimethoxybenzylamino)-[1]-benzothieno-[2,3-d]-pyrimidin-2-yl]-piperidin-4-carbonsäureethylester;
1-[5,6-Cyclopenteno-4-(3,4-dimethoxybenzylamino)-[1]-benzothieno-[2,3-d]-pyrimidin-2-yl]-piperidin-4-carbonsäureethylester;
1-[5,6-Cyclohepteno-4-(3,4-dimethoxybenzylamino)-[1]-benzothieno-[2,3-d]-pyrimidin-2-yl]-piperidin-4-carbonsäureethylester;
1-[6-Ethyl-4-(3,4-dimethoxybenzylamino)-thieno-[2,3-d]-pyrimidin-2-yl]-piperidin-4-carbonsäureethylester;
1-[6-Chlor-4-(3,4-dimethoxybenzylamino)-thieno-[2,3-d)-pyrimidin-2-yl]-piperidin-4-carbonsäureethylester;
1-[5-Chlor-6-methyl-4-(3,4-dimethoxybenzylamino)-thieno-[2,3-d]-pyrimidin-2-yl]-piperidin-4-carbonsäureethylester;
1-[6-Nitro-4-(3,4-dimethoxybenzylamino)-thieno-[2,3-d]-pyrimidin-2-yl]-piperidin-4-carbonsäureethylester;
1-[5,6-Dimethyl-4-(3,4-dimethoxybenzylamino)-thieno-[2,3-d]-pyrimidin-2-yl]-piperidin-4-carbonsäureethylester;
1-[6-Trifluormethyl-4-(3,4-dimethoxybenzylamino)-thieno-[2,3-d]-pyrimidin-2-yl]-piperidin-4-carbonsäureethylester;
1-[6-Methyl-4-(3,4-dimethoxybenzylamino)-thieno-[2,3-d]-pyrimidin-2-yl]-piperidin-4-carbonsäureethylester;
1-(6-Methyl-4-benzylamino-thieno-[2,3-d]-pyrimidin-2-yl)-piperidin-4-carbonsäureethylester;
1-(5-Methyl-4-benzylamino-thieno-[2,3-d]-pyrimidin-2-yl)-piperidin-4-carbonsäureethylester;
1-(5,6,7,8-Tetrahydro-4-benzylamino-[1]-benzothieno-[2,3-d]-pyrimidin-2-yl)-piperidin-4-carbonsäureethylester;
1-(5,6-Cyclopenteno-4-benzylamino-[1]-benzothieno-[2,3-d]-pyrimidin-2-yl)-piperidin-4-carbonsäureethylester;
1-(5,6-Cyclohepteno-4-benzylamino-[1]-benzothieno-[2,3-d]-pyrimidin-2-yl)-piperidin-4-carbonsäureethylester;
1-(6-Ethyl-4-benzylamino-thieno-[2,3-d]-pyrimidin-2-yl)-piperidin-4-carbonsäureethylester;
1-(6-Chlor-4-benzylamino-thieno-[2,3-d]-pyrimidin-2-yl)-piperidin-4-carbonsäureethylester;
1-(5-Chlor-6-methyl-4-benzylamino-thieno-[2,3-d]-pyrimidin-2-yl)-piperidin-4-carbonsäureethylester;
1-(6-Nitro-4-benzylamino-thieno-[2,3-d]-pyrimidin-2-yl)-piperidin-4-carbonsäureethylester;
1-(5,6-Dimethyl-4-benzylamino-thieno-[2,3-d]-pyrimidin-2-yl)-piperidin-4-carbonsäureethylester;
1-(6-Trifluormethyl-4-benzylamino-thieno-[2,3-d]-pyrimidin-2-yl)-piperidin-4-carbonsäureethylester,
1-[6-Methyl-4-(4-fluorbenzylamino)-thieno-[2,3-d]-pyrimidin-2-yl]-piperidin-4-carbonsäureethylester;
1-[5-Methyl-4-(4-fluorbenzylamino)-thieno-[2,3-d]-pyrimidin-2-yl]-piperidin-4-carbonsäureethylester;
1-[5,6,7,8-Tetrahydro-4-(4-fluorbenzylamino)-[1]-benzothieno-[2,3-d]-pyrimidin-2-yl]-piperidin-4-carbonsäureethylester;
1-[5,6-Cyclopenteno-4-[4-fluorbenzylamino)-[1]-benzothieno-[2,3-d]-pyrimidin-2-yl]-piperidin-4-carbonsäureethylester;
1-[5,6-Cyclohepteno-4-(4-fluorbenzylamino)-[1]-benzothieno-[2,3-d]-pyrimidin-2-yl]-piperidin-4-carbonsäureethylester;
1-[6-Ethyl-4-(4-fluorbenzylamino)-thieno-[2,3-d]-pyrimidin-2-yl]-piperidin-4-carbonsäureethylester;
1-[6-Chlor-4-(4-fluorbenzylamino)-thieno-[2,3-d]-pyrimidin-2-yl]-piperidin-4-carbonsäureethylester;
1-[5-Chlor-6-methyl-4-(4-fluorbenzylamino)-thieno-[2,3-d]-pyrimidin-2-yl]-piperidin-4-carbonsäureethylester;
1-[6-Nitro-4-(4-fluorbenzylamino)-thieno-[2,3-d]-pyrimidin-2-yl]-piperidin-4-carbonsäureethylester;
1-[5,6-Dimethyl-4-(4-fluorbenzylamino)-thieno-[2,3-d]-pyrimidin-2-yl]-piperidin-4-carbonsäureethylester;
1-[6-Trifluormethyl-4-(4-fluorbenzylamino)-thieno-[2,3-d]-pyrimidin-2-yl]-piperidin-4-carbonsäureethylester;
1-[6-Methyl-4-(3,4-dichlorbenzylamino)-thieno-[2,3-d]-pyrimidin-2-yl]-piperidin-4-carbonsäureethylester;
1-[5-Methyl-4-(3,4-dichlorbenzylamino)-thieno-[2,3-d]-pyrimidin-2-yl]-piperidin-4-carbonsäureethylester;
1-[5,6,7,8-Tetrahydro-4-(3,4-dichlorbenzylamino)-[1]-benzothieno-[2,3-d]-pyrimidin-2-yl]-piperidin-4-carbonsäureethylester;
1-[5,6-Cyclopenteno-4-(3,4-dichlorbenzylamino)-[1]-benzothieno-[2,3-d]-pyrimidin-2-yl]-piperidin-4-carbonsäureethylester;
1-[5,6-Cyclohepteno-4-(3,4-dichlorbenzylamino)-[1]-benzothieno-[2,3-d]-pyrimidin-2-yl]-piperidin-4-carbonsäureethylester;
1-[6-Ethyl-4-(3,4-dichlorbenzylamino)-thieno-[2,3-d]-pyrimidin-2-yl]-piperidin-4-carbonsäureethylester;
1-[6-Chlor-4-(3,4-dichlorbenzylamino)-thieno-[2,3-d]-pyrimidin-2-yl]-piperidin-4-carbonsäureethylester;
1-[5-Chlor-6-methyl-4-(3,4-dichlorbenzylamino)-thieno-[2,3-d]-pyrimidin-2-yl]-piperidin-4-carbonsäureethylester;
1-[6-Nitro-4-(3,4-dichlorbenzylamino)-thieno-[2,3-d]-pyrimidin-2-yl]-piperidin-4-carbonsäureethylester;
1-[5,6-Dimethyl-4-(3,4-dichlorbenzylamino)-thieno-[2,3-d]-pyrimidin-2-yl]-piperidin-4-carbonsäureethylester;
1-[6-Trifluormethyl-4-(3,4-dichlorbenzylamino)-thieno-[2,3-d]-pyrimidin-2-yl]-piperidin-4-carbonsäureethylester;
1-[6-Methyl-4-(3-nitrobenzylamino)-thieno-[2,3-d]-pyrimidin-2-yl]-piperidin-4-carbonsäureethylester;
1-[5-Methyl-4-(3-nitrobenzylamino)-thieno-[2,3-d]-pyrimidin-2-yl]-piperidin-4-carbonsäureethylester;
1-[5,6,7,8-Tetrahydro-4-(3-nitrobenzylamino)-[1]-benzothieno-[2,3-d]-pyrimidin-2-yl]-piperidin-4-carbonsäureethylester;
1-[5,6-Cyclopenteno-4-(3-nitrobenzylamino)-[1]-benzothieno-[2,3-d]-pyrimidin-2-yl]-piperidin-4-carbonsäureethylester;
1-[5,6-Cyclohepteno-4-(3-nitrobenzylamino)-[1]-benzothieno-[2,3-d]-pyrimidin-2-yl]-piperidin-4-carbonsäureethylester;
1-[6-Ethyl-4-(3-nitrobenzylamino)-thieno-[2,3-d]-pyrimidin-2-yl]-piperidin-4-carbonsäureethylester;
1-[6-Chlor-4-(3-nitrobenzylamino)-thieno-[2,3-d]-pyrimidin-2-yl]-piperidin-4-carbonsäureethylester;
1-[5-Chlor-6-methyl-4-(3-nitrobenzylamino)-thieno-[2,3-d]-pyrimidin-2-yl]-piperidin-4-carbonsäureethylester;
1-[6-Nitro-4-(3-nitrobenzylamino)-thieno-[2,3-d]-pyrimidin-2-yl]-piperidin-4-carbonsäureethylester;
1-[5,6-Dimethyl-4-(3-nitrobenzylamino)-thieno-[2,3-d]-pyrimidin-2-yl]-piperidin-4-carbonsäureethylester;
1-[6-Trifluormethyl-4-(3-nitrobenzylamino)-thieno-[2,3-d]-pyrimidin-2-yl]-piperidin-4-carbonsäureethylester;
1-[6-Methyl-4-(3,4-methylendioxyphenethylamino)-thieno-[2,3-d]-pyrimidin-2-yl]-piperidin-4-carbonsäureethylester;
1-[5-Methyl-4-(3,4-methylendioxyphenethylamino)-thieno-[2,3-d]-pyrimidin-2-yl]-piperidin-4-carbonsäureethylester;
1-[5,6,7,8-Tetrahydro-4-(3,4-methylendioxyphenethylamino)-[1]-benzothieno-[2,3-d]-pyrimidin-2-yl]-piperidin-4-carbonsäureethylester;
1-[5,6-Cyclopenteno-4-(3,4-methylendioxyphenethylamino)-[1]-benzothieno-[2,3-d]-pyrimidin-2-yl]-piperidin-4-carbonsäureethylester;
1-[5,6-Cyclohepteno-4-(3,4-methylendioxyphenethylamino)-[1]-benzothieno-[2,3-d]-pyrimidin-2-yl]-piperidin-4-carbonsäureethylester;
1-[6-Ethyl-4-(3,4-methylendioxyphenethylamino)-thieno-[2,3-d]-pyrimidin-2-yl]-piperidin-4-carbonsäureethylester;
1-[6-Chlor-4-(3,4-methylendioxyphenethylamino)-thieno-[2,3-d]-pyrimidin-2-yl]-piperidin-4-carbonsäureethylester;
1-[5-Chlor-6-methyl-4-(3,4-methylendioxyphenethylamino)-thieno-[2,3-d]-pyrimidin-2-yl]-piperidin-4-carbonsäureethylester;
1-[6-Nitro-4-(3,4-methylendioxyphenethylamino)-thieno-[2,3-d]-pyrimidin-2-yl]-piperidin-4-carbonsäureethylester;
1-[5,6-Dimethyl-4-(3,4-methylendioxyphenethylamino)-thieno-[2,3-d]-pyrimidin-2-yl]-piperidin-4-carbonsäureethylester;
1-[6-trifluormethyl-4-(3,4-methylendioxy-phenethylamino)-thieno-[2,3-d]-pyrimidin-2-yl]-piperidin-4-carbonsäureethylester;
1-[6-Methyl-4-(3,4-ethylendioxybenzylamino)-thieno-[2,3-d]-pyrimidin-2-yl]-piperidin-4-carbonsäureethylester;
1-[5-Methyl-4-(3,4-ethylendioxybenzylamino)-thieno-[2,3-d]-pyrimidin-2-yl]-piperidin-4-carbonsäureethylester;
1-[5,6,7,8-Tetrahydro-4-(3,4-ethylendioxybenzylamino)-[1]-benzothieno-[2,3-d]-pyrimidin-2-yl]-piperidin-4-carbonsäureethylester;
1-[5,6-Cyclopenteno-4-(3,4-ethylendioxybenzylamino)-[1]-benzothieno-[2,3-d]-pyrimidin-2-yl]-piperidin-4-carbonsäureethylester;
1-[5,6-Cyclohepteno-4-(3,4-ethylendioxybenzylamino)-[1]-benzothieno-[2,3-d]-pyrimidin-2-yl]-piperidin-4-carbonsäureethylester;
1-[6-Ethyl-4-(3,4-ethylendioxybenzylamino)-thieno-[2,3-d]-pyrimidin-2-yl]-piperidin-4-carbonsäureethylester;
1-[6-Chlor-4-(3,4-ethylendioxybenzylamino)-thieno-[2,3-d]-pyrimidin-2-yl]-piperidin-4-carbonsäureethylester;
1-[5-Chlor-6-methyl-4-(3,4-ethylendioxybenzylamino)-thieno-[2,3-d]-pyrimidin-2-yl]-piperidin-4-carbonsäureethylester;
1-[6-Nitro-4-(3,4-ethylendioxybenzylamino)-thieno-[2,3-d]-pyrimidin-2-yl]-piperidin-4-carbonsäureethylester;
1-[5,6-Dimethyl-4-(3,4-ethylendioxybenzylamino)-thieno-[2,3-d]-pyrimidin-2-yl]-piperidin-4-carbonsäureethylester;
1-[6-Trifluormethyl-4-(3,4-ethylendioxy-benzylamino)-thieno-[2,3-d]-pyrimidin-2-yl]-piperidin-4-carbonsäureethylester.

### Beispiel 3

0,5 g 1-[6-Methyl-4-(3,4-methylendioxybenzylamino)-thieno-[2,3-d]-pyrimidin-2-yl]-piperidin-4-carbonsäureethylester wird in 70 ml Methanol gelöst und nach Zugabe von 30 ml 2N NaOH 4 Stunden bei 50° gerührt. Nach Entfernen des Lösungsmittels und Waschen mit kaltem Wasser erhält man 1,5 g des Natriumsalzes der 1-[6-Methyl-4-(3,4-methylendioxybenzylamino)-thieno-[2,3-d]-pyrimidin-2-yl]-piperidin-4-carbonsäure, F. 272°.

Analog erhält man aus den unter Beispiel 2 aufgeführten Estem die nachstehenden Carbonsäuren:
1-[5-Methyl-4-(3,4-methylendioxybenzylamino)-thieno-[2,3-d]-pyrimidin-2-yl]-piperidin-4-carbonsäure;
1-[5,6,7,8-Tetrahydro-4-(3,4-methylendioxybenzylamino)-[1]-benzothieno-[2,3-d]-pyrimidin-2-yl]-piperidin-4-carbonsäure, Monohydrat, amorph (Zersetzung);
1-[5,6-Cyclopenteno-4-(3,4-methylendioxybenzylamino)-[1]-benzothieno-[2,3-d]-pyrimidin-2-yl]-piperidin-4-carbonsäure, F. >250°;
1-[5,6-Cyclohepteno-4-(3,4-methylendioxybenzylamino)-[1]-benzothieno-[2,3-d]-pyrimidin-2-yl]-piperidin-4-Carbonsäure, F. 217°;
1-[6-Ethyl-4-(3,4-methylendioxybenzylamino)-thieno-[2,3-d]-pyrimidin-2-yl]-piperidin-4-carbonsäure;
1-[6-Chlor-4-(3,4-methylendioxybenzylamino)-thieno-[2,3-d]-pyrimidin-2-yl]-piperidin-4-carbonsäure;
1-[5-Chlor-6-methyl-4-(3,4-methylendioxybenzylamino)-thieno-[2,3-d]-pyrimidin-2-yl]-piperidin-4-carbonsäure, amorph (Zersetzung);
1-[6-Nitro-4-(3,4-methylendioxybenzylamino)-thieno-[2,3-d]-pyrimidin-2-yl]-piperidin-4-carbonsäure, amorph (Zersetzung);
1-[5,6-Dimethyl-4-(3,4-methylendioxybenzylamino)-thieno-[2,3-d]-pyrimidin-2-yl]-piperidin-4-carbonsäure;
1 -[6-Trifluormethyl-4-(3,4-methylendioxybenzylamino)-thieno-[2,3-d]-pyrimidin-2-yl]-piperidin-4-carbonsäure;
1-[6-Methyl-4-(3-chlor-4-methoxybenzylamino)-thieno-[2,3-d]-pyrimidin-2-yl]-piperidin-4-carbonsäure;
1-[5-Methyl-4-(3-chlor-4-methoxybenzylamino)-thieno-[2,3-d]-pyrimidin-2-yl]-piperidin-4-carbonsäure;
1-[5,6,7,8-Tetrahydro-4-(3-chlor-4-methoxybenzylamino)-[1]-benzothieno-[2,3-d]-pyrimidin-2-yl]-piperidin-4-carbonsäure, Natriumsalz, F. 213°;
1-[5,6-Cyclopenteno-4-(3-chlor-4-methoxybenzylamino)-[1]-benzothieno-[2,3-d]-pyrimidin-2-yl]-piperidin-4-carbonsäure, Natriumsalz, F. >250°; Kaliumsalz F. >250°;
1-[5,6-Cyclohepteno-4-(3-chlor-4-methoxybenzylamino)-[1]-benzothieno-[2,3-d]-pyrimidin-2-yl]-piperidin-4-carbonsäure;
1-[6-Ethyl-4-(3-chlor-4-methoxybenzylamino)-thieno-[2,3-d]-pyrimidin-2-yl]-piperidin-4-carbonsäure;
1-[6-Chlor-4-(3-chlor-4-methoxybenzylamino)-thieno-[2,3-d]-pyrimidin-2-yl]-piperidin-4-carbonsäure;
1-[5-Chlor-6-methyl-4-(3-chlor-4-methoxybenzylamino)-thieno-[2,3-d]-pyrimidin-2-yl]-piperidin-4-carbonsäure, Natriumsalz, F. >250°;
1-[6-Nitro-4-(3-chlor-4-methoxybenzylamino)-thieno-[2,3-d]-pyrimidin-2-yl]-piperidin-4-carbonsäure;
1-[5,6-Dimethyl-4-(3-chlor-4-methoxybenzylamino)-thieno-[2,3-d]-pyrimidin-2-yl]-piperidin-4-carbonsäure, Natriumsalz, amorph;
1-[6-Trifluormethyl-4-(3-chlor-4-methoxybenzylamino)-thieno-[2,3-d]-pyrimidin-2-yl]-piperidin-4-carbonsäure;
1-[6-Methyl-4-(3,4-dimethoxybenzylamino)-thieno-[2,3-d]-pyrimidin-2-yl]-piperidin-4-carbonsäure;
1-[5-Methyl-4-(3,4-dimethoxybenzylamino)-thieno-[2,3-d]-pyrimidin-2-yl]-piperidin-4-carbonsäure;
1-[5,6,7,8-Tetrahydro-4-(3,4-dimethoxybenzylamino)-[1]-benzothieno-[2,3-d]-pyrimidin-2-yl]-piperidin-4-carbonsäure;
1-[5,6-Cyclopenteno-4-(3,4-dimethoxybenzylamino)-[1]-benzothieno-[2,3-d]-pyrimidin-2-yl]-piperidin-4-carbonsäure;
1-[5,6-Cyclohepteno-4-(3,4-dimethoxybenzylamino)-[1]-benzothieno-[2,3-d]-pyrimidin-2-yl]-piperidin-4-carbonsäure;
1-[6-Ethyl-4-(3,4-dimethoxybenzylamino)-thieno-[2,3-d]-pyrimidin-2-yl]-piperidin-4-carbonsäure;
1-[6-Chlor-4-(3,4-dimethoxybenzylamino)-thieno-[2,3-d]-pyrimidin-2-yl]-piperidin-4-carbonsäure;
1-[5-Chlor-6-methyl-4-(3,4-dimethoxybenzylamino)-thieno-[2,3-d]-pyrimidin-2-yl]-piperidin-4-carbonsäure;
1-[6-Nitro-4-(3,4-dimethoxybenzylamino)-thieno-[2,3-d]-pyrimidin-2-yl]-piperidin-4-carbonsäure;
1-[5,6-Dimethyl-4-(3,4-dimethoxybenzylamino)-thieno-[2,3-d]-pyrimidin-2-yl]-piperidin-4-carbonsäure;
1-[6-Trifluormethyl-4-(3,4-dimethoxybenzylamino)-thieno-[2,3-d]-pyrimidin-2-yl]-piperidin-4-carbonsäure;
1-[6-Methyl-4-(3,4-dimethoxybenzylamino)-thieno-[2,3-d]-pyrimidin-2-yl]-piperidin-4-carbonsäure;
1-(6-Methyl-4-benzylamino-thieno-[2,3-d]-pyrimidin-2-yl)-piperidin-4-carbonsäure, F. 203°;
1-(5-Methyl-4-benzylamino-thieno-[2,3-d]-pyrimidin-2-yl)-piperidin-4-carbonsäure;
1-(5,6,7,8-Tetrahydro-4-benzylamino-[1]-benzothieno-[2,3-d]-pyrimidin-2-yl)-piperidin-4-carbonsäure;
1-(5,6-Cyclopenteno-4-benzylamino-[1]-benzothieno-[2,3-d]-pyrimidin-2-yl)-piperidin-4-carbonsäure;
1-(5,6-Cyclohepteno-4-benzylamino-[1]-benzothieno-[2,3-d]-pyrimidin-2-yl)-piperidin-4-carbonsäure, F. 257°;
1-(6-Ethyl-4-benzylamino-thieno-[2,3-d]-pyrimidin-2-yl)-piperidin-4-carbonsäure, Natriumsalz, amorph;
1-(6-Chlor-4-benzylamino-thieno-[2,3-d]-pyrimidin-2-yl)-piperidin-4-carbonsäure;
1-(5-Chlor-6-methyl-4-benzylamino-thieno-[2,3-d]-pyrimidin-2-yl)-piperidin-4-carbonsäure;
1-(6-Nitro-4-benzylamino-thieno-[2,3-d]-pyrimidin-2-yl)-piperidin-4-carbonsäure;
1-(5,6-Dimethyl-4-benzylamino-thieno-[2,3-d]-pyrimidin-2-yl)-piperidin-4-carbonsäure;
1-(6-Trifluormethyl-4-benzylamino-thieno-[2,3-d]-pyrimidin-2-yl)-piperidin-4-carbonsäure;
1-[6-Methyl-4-(4-fluorbenzylamino)-thieno-[2,3-d]-pyrimidin-2-yl]-piperidin-4-carbonsäure;
1-[5-Methyl-4-(4-fluorbenzylamino)-thieno-[2,3-d]-pyrimidin-2-yl]-piperidin-4-carbonsäure;
1-[5,6,7,8-Tetrahydro-4-(4-fluorbenzylamino)-[1]-benzothieno-[2,3-d]-pyrimidin-2-yl]-piperidin-4-carbonsäure, Natriumsalz, F. 279°;
1-[5,6-Cyclopenteno-4-(4-fluorbenzylamino)-[1]-benzothieno-[2,3-d]-pyrimidin-2-yl]-piperidin-4-carbonsäure;
1-[5,6-Cyclohepteno-4-(4-fluorbenzylamino)-[1]-benzothieno-[2,3-d]-pyrimidin-2-yl]-piperidin-4-carbonsäure;
1-[6-Ethyl-4-(4-fluorbenzylamino)-thieno-[2,3-d]-pyrimidin-2-yl]-piperidin-4-carbonsäure;
1-[6-Chlor-4-(4-fluorbenzylamino)-thieno-[2,3-d]-pyrimidin-2-yl]-piperidin-4-carbonsäure;
1-[5-Chlor-6-methyl-4-(4-fluorbenzylamino)-thieno-[2,3-d]-pyrimidin-2-yl]-piperidin-4-carbonsäure;
1-[6-Nitro-4-(4-fluorbenzylamino)-thieno-[2,3-d]-pyrimidin-2-yl]-piperidin-4-carbonsäure;
1-[5,6-Dimethyl-4-(4-fluorbenzylamino)-thieno-[2,3-d]-pyrimidin-2-yl]-piperidin-4-carbonsäure;
1-[6-Trifluormethyl-4-(4-fluorbenzylamino)-thieno-[2,3-d]-pyrimidin-2-yl]-piperidin-4-carbonsäure;
1-[6-Methyl-4-(3,4-dichlorbenzylamino)-thieno-[2,3-d]-pyrimidin-2-yl]-piperidin-4-carbonsäure;
1-[5-Methyl-4-(3,4-dichlorbenzylamino)-thieno-[2,3-d]-pyrimidin-2-yl]-piperidin-4-carbonsäure;
1-[5,6,7,8-Tetrahydro-4-(3,4-dichlorbenzylamino)-[1]-benzothieno-[2,3-d]-pyrimidin-2-yl]-piperidin-4-carbonsäure;
1-[5,6-Cyclopenteno-4-(3,4-dichlorbenzylamino)-[1]-benzothieno-[2,3-d]-pyrimidin-2-yl]-piperidin-4-carbonsäure;
1-[5,6-Cyclohepteno-4-(3,4-dichlorbenzylamino)-[1]-benzothieno-[2,3-d]-pyrimidin-2-yl]-piperidin-4-carbonsäure;
1-[6-Ethyl-4-(3,4-dichlorbenzylamino)-thieno-[2,3-d]-pyrimidin-2-yl]-piperidin-4-carbonsäure;
1-[6-Chlor-4-(3,4-dichlorbenzylamino)-thieno-[2,3-d]-pyrimidin-2-yl]-piperidin-4-carbonsäure;
1-[5-Chlor-6-methyl-4-(3,4-dichlorbenzylamino)-thieno-[2,3-d]-pyrimidin-2-yl]-piperidin-4-carbonsäure;
1-[6-Nitro-4-(3,4-dichlorbenzylamino)-thieno-[2,3-d]-pyrimidin-2-yl]-piperidin-4-carbonsäure;
1-[5,6-Dimethyl-4-(3,4-dichlorbenzylamino)-thieno-[2,3-d]-pyrimidin-2-yl]-piperidin-4-carbonsäure;
1-[6-Trifluormethyl-4-(3,4-dichlorbenzylamino)-thieno-[2,3-d]-pyrimidin-2-yl]-piperidin-4-carbonsäure;
1-[6-Methyl-4-(3-nitrobenzylamino)-thieno-[2,3-d]-pyrimidin-2-yl]-piperidin-4-carbonsäure;
1-[5-Methyl-4-(3-nitrobenzylamino)-thieno-[2,3-d]-pyrimidin-2-yl]-piperidin-4-carbonsäure;
1-[5,6,7,8-Tetrahydro-4-(3-nitrobenzylamino)-[1]-benzothieno-[2,3-d]-pyrimidin-2-yl]-piperidin-4-carbonsäure;
1-[5,6-Cyclopenteno-4-(3-nitrobenzylamino)-[1]-benzothieno-[2,3-d]-pyrimidin-2-yl]-piperidin-4-carbonsäure;
1-[5,6-Cyclohepteno-4-(3-nitrobenzylamino)-[1]-benzothieno-[2,3-d]-pyrimidin-2-yl]-piperidin-4-carbonsäure;
1-[6-Ethyl-4-(3-nitrobenzylamino)-thieno-[2,3-d]-pyrimidin-2-yl]-piperidin-4-carbonsäure;
1-[6-Chlor-4-(3-nitrobenzylamino)-thieno-[2,3-d]-pyrimidin-2-yl]-piperidin-4-carbonsäure;
1-[5-Chlor-6-methyl-4-(3-nitrobenzylamino)-thieno-[2,3-d]-pyrimidin-2-yl]-piperidin-4-carbonsäure;
1-[6-Nitro-4-(3-nitrobenzylamino)-thieno-[2,3-d]-pyrimidin-2-yl]-piperidin-4-carbonsäure;
1-[5,6-Dimethyl-4-(3-nitrobenzylamino)-thieno-[2,3-d]-pyrimidin-2-yl]-piperidin-4-carbonsäure;
1-[6-Trifluormethyl-4-(3-nitrobenzylamino)-thieno-[2,3-d]-pyrimidin-2-yl]-piperidin-4-carbonsäure;
1-[6-Methyl-4-(3,4-methylendioxyphenethylamino)-thieno-[2,3-d]-pyrimidin-2-yl]-piperidin-4-carbonsäure;
1-[5-Methyl-4-(3,4-methylendioxyphenethylamino)-thieno-[2,3-d]-pyrimidin-2-yl]-piperidin-4-carbonsäure;
1-[5,6,7,8-Tetrahydro-4-(3,4-methylendioxyphenethylamino)-[1]-benzothieno-[2,3-d]-pyrimidin-2-yl]-piperidin-4-carbonsäure;
1-[5,6-Cyclopenteno-4-(3,4-methylendioxyphenethylamino)-[1]-benzothieno-[2,3-d]-pyrimidin-2-yl]-piperidin-4-carbonsäure;
1-[5,6-Cyclohepteno-4-(3,4-methylendioxyphenethylamino)-[1]-benzothieno-[2,3-d]-pyrimidin-2-yl]-piperidin-4-carbonsäure;
1-[6-Ethyl-4-(3,4-methylendioxyphenethylamino)-thieno-[2,3-d]-pyrimidin-2-yl]-piperidin-4-carbonsäure;
1-[6-Chlor-4-(3,4-methylendioxyphenethylamino)-thieno-[2,3-d]-pyrimidin-2-yl]-piperidin-4-carbonsäure;
1-[5-Chlor-6-methyl-4-(3,4-methylendioxyphenethylamino)-thieno-[2,3-d]-pyrimidin-2-yl]-piperidin-4-carbonsäure;
1-[6-Nitro-4-(3,4-methylendioxyphenethylamino)-thieno-[2,3-d]-pyrimidin-2-yl]-piperidin-4-carbonsäure;
1-[5,6-Dimethyl-4-(3,4-methylendioxyphenethylamino)-thieno-[2,3-d]-pyrimidin-2-yl]-piperidin-4-carbonsäure;
1-[6-Trifluormethyl-4-(3,4-methylendioxyphenethylamino)-thieno-[2,3-d]-pyrimidin-2-yl]-piperidin-4-carbonsäure;
1-[6-Methyl-4-(3,4-ethylendioxybenzylamino)-thieno-[2,3-d]-pyrimidin-2-yl]-piperidin-4-carbonsäure;
1-[5-Methyl-4-(3,4-ethylendioxybenzylamino)-thieno-[2,3-d]-pyrimidin-2-yl]-piperidin-4-carbonsäure;
1-[5,6,7,8-Tetrahydro-4-(3,4-ethylendioxybenzylamino)-[1]-benzothieno-[2,3-d]-pyrimidin-2-yl]-piperidin-4-carbonsäure;
1-[5,6-Cyclopenteno-4-(3,4-ethylendioxybenzylamino)-[1]-benzothieno-[2,3-d]-pyrimidin-2-yl]-piperidin-4-carbonsäure;
1-[5,6-Cyclohepteno-4-(3,4-ethylendioxybenzylamino)-[1]-benzothieno-[2,3-d]-pyrimidin-2-yl]-piperidin-4-carbonsäure;
1-[6-Ethyl-4-(3,4-ethylendioxybenzylamino)-thieno-[2,3-d]-pyrimidin-2-yl]-piperidin-4-carbonsäure;
1-[6-Chlor-4-(3,4-ethylendioxybenzylamino)-thieno-[2,3-d]-pyrimidin-2-yl]-piperidin-4-carbonsäure;
1-[5-Chlor-6-methyl-4-(3,4-ethylendioxybenzylamino)-thieno-[2,3-d]-pyrimidin-2-yl]-piperidin-4-carbonsäure;
1-[6-Nitro-4-(3,4-ethylendioxybenzylamino)-thieno-[2,3-d]-pyrimidin-2-yl]-piperidin-4-carbonsäure;
1-[5,6-Dimethyl-4-(3,4-ethylendioxybenzylamino)-thieno-[2,3-d]-pyrimidin-2-yl]-piperidin-4-carbonsäure;
1-[6-Trifluormethyl-4-(3,4-ethylendioxy-benzylamino)-thieno-[2,3-d]-pyrimidin-2-yl]-piperidin-4-carbonsäure.

### Beispiel 4

5 g 2-Amino-5-methyl-3-ethoxycarbonyl-thiophen wird mit 2,7 g 4-Cyanbenzoesäuremethylester in 40 ml Dioxan gelöst. Anschließend wird für 5 Stunden gasförmiges HCl durch die Lösung geleitet. Nach üblicher Aufarbeitung erhält man 6 g 4-(3,4-Dihydro-4-oxo-6-methyl-thieno-[2,3-d]-pyrimidin-2-yl)-benzoesäuremethylester.
Der Ersatz der Carbonylgruppe durch Cl unter Ausbildung des aromatischen Pyrimidinrings erfolgt unter Standardbedingungen.
Eine Mischung aus 18 ml POCl₃ mit 6 g 4-(3,4-Dihydro-4-oxo-6-methylthieno-[2,3-d]-pyrimidin-2-yl)-benzoesäuremethylester unter Zusatz von 1,8 ml N,N-Dimethylanilin wird 4 Stunden gekocht. Nach üblicher Aufarbeitung erhält man 5 g 4-(4-Chlor-6-methyl-thieno-[2,3-d]-pyrimidin-2-yl)-benzoesäuremethylester.

Analog erhält man durch Umsetzung von 4-Cyanbenzoesäuremethylester und anschließender Reaktion mit POCl₃
aus 2-Amino-4-methyl-3-ethoxycarbonyl-thiophen
   4-(4-Chlor-5-methyl-thieno-[2,3-d]-pyrimidin-2yl)-benzoesäuremethylester;
aus 2-Amino-4,5,6,7-tetrahydro-3-ethoxycarbonyl-benzothiophen
   4-(4-Chlor-5,6,7,8-tetrahydro-[1]-benzothieno-[2,3-d]-pyrimidin-2-yl)-benzoesäuremethylester;
aus 2-Amino-4,5-cyclopenteno-3-ethoxycarbonyl-thiophen
   4-(4-Chlor-5,6-cyclopenteno-thieno-[2,3-d]-pyrimidin-2-yl)-benzoesäuremethylester;
aus 2-Amino-4,5-cyclohepteno-3-ethoxycarbonyl-thiophen
   4-(4-Chlor-5,6-cyclohepteno-thieno-[2,3-d]-pyrimidin-2-yl)-benzoesäuremethylester;
aus 2-Amino-5-ethyl-3-ethoxycarbonyl-thiophen
   4-(4-Chlor-6-ethyl-thieno-[2,3-d]-pyrimidin-2-yl)-benzoesäuremethylester;
aus 2-Amino-5-propyl-3-ethoxycarbonyl-thiophen
   4-(4-Chlor-6-propyl-thieno-[2,3-d]-pyrimidin-2-yl)-benzoesäuremethylester;
aus 2-Amino-5-chlor-3-ethoxycarbonyl-thiophen
   4-(4-Chlor-6-chlor-thieno-[2,3-d]-pyrimidin-2-yl)-benzoesäuremethylester;
aus 2-Amino-4-chlor-5-methyl-3-ethoxycarbonyl-thiophen
   4-(4-Chlor-5-chlor-6-methyl-thieno-[2,3-d]-pyrimidin-2-yl)-benzoesäuremethylester;
aus 2-Amino-5-nitro-3-ethoxycarbonyl-thiophen
   4-(4-Chlor-6-nitro-thieno-[2,3-d)-pyrimidin-2-yl)-benzoesäuremethylester;
aus 2-Amino-4,5-dimethyl-3-ethoxycarbonyl-thiophen
   4-(4-Chlor-5,6-dimethyl-thieno-[2,3-d]-pyrimidin-2-yl)-benzoesäuremethylester;
aus 2-Amino-5-trifluormethyl-3-ethoxycarbonyl-thiophen
   4-(4-Chlor-6-trifluormethyl-thieno-[2,3-d]-pyrimidin-2-yl)-benzoesäuremethylester.

### Beispiel 5

Analog Beispiel 1 erhält man durch Umsetzung von 3,4-Methylendioxybenzylamin
mit 4-(4-Chlor-6-methyl-thieno-[2,3-d]-pyrimidin-2-yl)-benzoesäuremethylester
   4-[4-(3,4-Methylendioxybenzylamino)-6-methyl-thieno-[2,3-d]-pyrimidin-2-yl]-benzoesäuremethylester;
mit 4-(4-Chlor-5-methyl-thieno-[2,3-d]-pyrimidin-2yl)-benzoesäuremethylester
   4-[4-(3,4-Methylendioxybenzylamino)-5-methyl-thieno-[2,3-d]-pyrimidin-2-yl]-benzoesäuremethylester;
mit 4-(4-Chlor-5,6,7,8-tetrahydro-[1]-benzothieno-[2,3-d]-pyrimidin-2-yl)-benzoesäuremethylester
   4-[4-(3,4-Methylendioxybenzylamino)-5,6,7,8-tetrahydro-[1]-benzothieno-[2,3-d]-pyrimidin-2-yl]-benzoesäuremethylester, F. 198°;
mit 4-(4-Chlor-5,6-cyclopenteno-thieno-[2,3-d]-pyrimidin-2-yl)-benzoesäuremethylester
   4-[4-(3,4-Methylendioxybenzylamino)-5,6-cyclopenteno-thieno-[2,3-d]-pyrimidin-2-yl]-benzoesäuremethylester;
mit 4-(4-Chlor-5,6-cyclohepteno-thieno-[2,3-d]-pyrimidin-2-yl)-benzoesäuremethylester
   4-[4-(3,4-Methylendioxybenzylamino)-5,6-cyclohepteno-thieno-[2,3-d]-pyrimidin-2-yl]-benzoesäuremethylester;
mit 4-(4-Chlor-6-ethyl-thieno-[2,3-d]-pyrimidin-2-yl)-benzoesäuremethylester
   4-[4-(3,4-Methylendioxybenzylamino)-6-ethyl-thieno-[2,3-d]-pyrimidin-2-yl]-benzoesäuremethylester;
mit 4-(4-Chlor-6-propyl-thieno-[2,3-d]-pyrimidin-2-yl)-benzoesäuremethylester
   4-[4-(3,4-Methylendioxybenzylamino)-6-propyl-thieno-[2,3-d]-pyrimidin-2-yl]-benzoesäuremethylester
mit 4-(4-Chlor-6-chlor-thieno-[2,3-d]-pyrimidin-2-yl)-benzoesäuremethylester
   4-[4-(3,4-Methylendioxybenzylamino)-6-chlor-thieno-[2,3-d]-pyrimidin-2-yl]-benzoesäuremethylester;
mit 4-(4-Chlor-5-chlor-6-methyl-thieno-[2,3-d]-pyrimidin-2-yl)-benzoesäuremethylester
   4-[4-(3,4-Methylendioxybenzylamino)-5-chlor-6-methyl-thieno-[2,3-d]-pyrimidin-2-yl]-benzoesäuremethylester;
mit 4-(4-Chlor-6-nitro-thieno-[2,3-d]-pyrimidin-2-yl)-benzoesäuremethylester
   4-[4-(3,4-Methylendioxybenzylamino)-6-nitro-thieno-[2,3-d]-pyrimidin-2-yl]-benzoesäuremethylester;
mit 4-(4-Chlor-5,6-dimethyl-thieno-[2,3-d]-pyrimidin-2-yl)-benzoesäuremethylester
   4-[4-(3,4-Methylendioxybenzylamino)-5,6-dimethyl-thieno-[2,3-d]-pyrimidin-2-yl]-benzoesäuremethylester;
mit 4-(4-Chlor-6-trifluormethyl-thieno-[2,3-d]-pyrimidin-2-yl)-benzoesäuremethylester
   4-[4-(3,4-Methylendioxybenzylamino)-6-trifluormethyl-thieno-[2,3-d]-pyrimidin-2-yl]-benzoesäuremethylester.

Analog erhält man durch Umsetzung von 3-Chlor-4-methoxy-benzylamin
mit 4-(4-Chlor-6-methyl-thieno-[2,3-d]-pyrimidin-2-yl)-benzoesäuremethylester
   4-[4-(3-Chlor-4-methoxybenzylamino)-6-methyl-thieno-[2,3-d]-pyrimidin-2-yl]-benzoesäuremethylester;
mit 4-(4-Chlor-5-methyl-thieno-[2,3-d]-pyrimidin-2yl)-benzoesäuremethylester
   4-[4-(3-Chlor-4-methoxybenzylamino)-5-methyl-thieno-[2,3-d]-pyrimidin-2-yl]-benzoesäuremethylester;
mit 4-(4-Chlor-5,6,7,8-tetrahydro-[1]-benzothieno-[2,3-d]-pyrimidin-2-yl)-benzoesäuremethylester
   4-[4-(3-Chlor-4-methoxybenzylamino)-5,6,7,8-tetrahydro-[1]-benzothieno-[2,3-d]-pyrimidin-2-yl]-benzoesäuremethylester;
mit 4-(4-Chlor-5,6-cyclopenteno-thieno-[2,3-d]-pyrimidin-2-yl)-benzoesäuremethylester
   4-[4-(3-Chlor-4-methoxybenzylamino)-5,6-cyclopenteno-thieno-[2,3-d]-pyrimidin-2-yl]-benzoesäuremethylester;
mit 4-(4-Chlor-5,6-cyclohepteno-thieno-[2,3-d]-pyrimidin-2-yl)-benzoesäuremethylester
   4-[4-(3-Chlor-4-methoxybenzylamino)-5,6-cyclohepteno-thieno-[2,3-d]-pyrimidin-2-yl]-benzoesäuremethylester;
mit 4-(4-Chlor-6-ethyl-thieno-[2,3-d]-pyrimidin-2-yl)-benzoesäuremethylester
   4-[4-(3-Chlor-4-methoxybenzylamino)-6-ethyl-thieno-[2,3-d]-pyrimidin-2-yl]-benzoesäuremethylester;
mit 4-(4-Chlor-6-chlor-thieno-[2,3-d]-pyrimidin-2-yl)-benzoesäuremethylester
   4-[4-(3-Chlor-4-methoxybenzylamino)-6-chlor-thieno-[2,3-d]-pyrimidin-2-yl]-benzoesäuremethylester;
mit 4-(4-Chlor-5-chlor-6-methyl-thieno-[2,3-d]-pyrimidin-2-yl)-benzoesäuremethylester
   4-[4-(3-Chlor-4-methoxybenzylamino)-5-chlor-6-methyl-thieno-[2,3-d]-pyrimidin-2-yl]-benzoesäuremethylester;
mit 4-(4-Chlor-6-nitro-thieno-[2,3-d]-pyrimidin-2-yl)-benzoesäuremethylester
   4-[4-(3-Chlor-4-methoxybenzylamino)-6-nitro-thieno-[2,3-d]-pyrimidin-2-yl]-benzoesäuremethylester;
mit 4-(4-Chlor-5,6-dimethyl-thieno-[2,3-d]-pyrimidin-2-yl)-benzoesäuremethylester
   4-[4-(3-Chlor-4-methoxybenzylamino)-5,6-dimethyl-thieno-[2,3-d]-pyrimidin-2-yl]-benzoesäuremethylester;
mit 4-(4-Chlor-6-trifluormethyl-thieno-[2,3-d]-pyrimidin-2-yl)-benzoesäuremethylester
   4-[4-(3-Chlor-4-methoxybenzylamino)-6-trifluormethyl-thieno-[2,3-d]-pyrimidin-2-yl]-benzoesäuremethylester.

Analog erhält man durch Umsetzung von 3,4-Dimethoxy-benzylamin
mit 4-(4-Chlor-6-methyl-thieno-[2,3-d]-pyrimidin-2-yl)-benzoesäuremethylester
   4-[4-(3,4-Dimethoxybenzylamino)-6-methyl-thieno-[2,3-d]-pyrimidin-2-yl]-benzoesäuremethylester;
mit 4-(4-Chlor-5-methyl-thieno-[2,3-d]-pyrimidin-2yl)-benzoesäuremethylester
   4-[4-(3,4-Dimethoxybenzylamino)-5-methyl-thieno-[2,3-d]-pyrimidin-2-yl]-benzoesäuremethylester;
mit 4-(4-Chlor-5,8,7,8-tetrahydro-[1]-benzothieno-[2,3-d]-pyrimidin-2-yl)-benzoesäuremethylester
   4-[4-(3,4-Dimethoxybenzylamino)-5,6,7,8-tetrahydro-[1]-benzothieno-[2,3-d]-pyrimidin-2-yl]-benzoesäuremethylester;
mit 4-(4-Chlor-5,6-cyclopenteno-thieno-[2,3-d]-pyrimidin-2-yl)-benzoesäuremethylester
   4-[4-(3,4-Dimethoxybenzylamino)-5,6-cyclopenteno-thieno-[2,3-d]-pyrimidin-2-yl]-benzoesäuremethylester;
mit 4-(4-Chlor-5,6-cyclohepteno-thieno-[2,3-d]-pyrimidin-2-yl)-benzoesäuremethylester
   4-[4-(3,4-Dimethoxybenzylamino)-5,6-cyclohepteno-thieno-[2,3-d]-pyrimidin-2-yl]-benzoesäuremethylester;
mit 4-(4-Chlor-6-ethyl-thieno-[2,3-d]-pyrimidin-2-yl)-benzoesäuremethylester
   4-[4-(3,4-Dimethoxybenzylamino)-6-ethyl-thieno-[2,3-d]-pyrimidin-2-yl]-benzoesäuremethylester;
mit 4-(4-Chlor-6-chlor-thieno-[2,3-d]-pyrimidin-2-yl)-benzoesäuremethylester
   4-[4-(3,4-Dimethoxybenzylamino)-6-chlor-thieno-[2,3-d]-pyrimidin-2-yl]-benzoesäuremethylester;
mit 4-(4-Chlor-5-chlor-6-methyl-thieno-[2,3-d]-pyrimidin-2-yl)-benzoesäuremethylester
   4-[4-(3,4-Dimethoxybenzylamino)-5-chlor-6-methyl-thieno-[2,3-d]-pyrimidin-2-yl]-benzoesäuremethylester;
mit 4-(4-Chlor-6-nitro-thieno-[2,3-d]-pyrimidin-2-yl)-benzoesäuremethylester
   4-[4-(3,4-Dimethoxybenzylamino)-6-nitro-thieno-[2,3-d]-pyrimidin-2-yl]-benzoesäuremethylester;
mit 4-(4-Chlor-5,6-dimethyl-thieno-[2,3-d]-pyrimidin-2-yl)-benzoesäuremethylester
   4-[4-(3,4-Dimethoxybenzylamino)-5,6-dimethyl-thieno-[2,3-d]-pyrimidin-2-yl]-benzoesäuremethylester;
mit 4-(4-Chlor-6-trifluormethyl-thieno-[2,3-d]-pyrimidin-2-yl)-benzoesäuremethylester
   4-[4-(3,4-Dimethoxybenzylamino)-6-trifluormethyl-thieno-[2,3-d]-pyrimidin-2-yl]-benzoesäuremethylester.

Analog erhält man durch Umsetzung von Benzylamin
mit 4-(4-Chlor-6-methyl-thieno-[2,3-d]-pyrimidin-2-yl)-benzoesäuremethylester
   4-(4-Benzylamino-6-methyl-thieno-[2,3-d]-pyrimidin-2-yl)-benzoesäuremethylester;
mit 4-(4-Chlor-5-methyl-thieno-[2,3-d]-pyrimidin-2yl)-benzoesäuremethylester
   4-(4-Benzylamino-5-methyl-thieno-[2,3-d]-pyrimidin-2-yl)-benzoesäuremethylester;
mit 4-(4-Chlor-5,6,7,8-tetrahydro-[1]-benzothieno-[2,3-d]-pyrimidin-2-yl)-benzoesäuremethylester
   4-(4-Benzylamino-5,6,7,8-tetrahydro-[1]-benzothieno-[2,3-d]-pyrimidin-2-yl)-benzoesäuremethylester;
mit 4-(4-Chlor-5,6-cyclopenteno-thieno-[2,3-d]-pyrimidin-2-yl)-benzoesäuremethylester
   4-(4-Benzylamino-5,6-cyclopenteno-thieno-[2,3-d]-pyrimidin-2-yl)-benzoesäuremethylester;
mit 4-(4-Chlor-5,6-cyclohepteno-thieno-[2,3-d]-pyrimidin-2-yl)-benzoesäuremethylester
   4-(4-Benzylamino)-5,6-cyclohepteno-thieno-[2,3-d]-pyrimidin-2-yl)-benzoesäuremethylester;
mit 4-(4-Chlor-6-ethyl-thieno-[2,3-d]-pyrimidin-2-yl)-benzoesäuremethylester
   4-(4-Benzylamino-6-ethyl-thieno-[2,3-d]-pyrimidin-2-yl)-benzoesäuremethylester;
mit 4-(4-Chlor-6-Chlor-thieno-[2,3-d]-pyrimidin-2-yl)-benzoesäuremethylester
   4-(4-Benzylamino-6-chlor-thieno-[2,3-d]-pyrimidin-2-yl)-benzoesäuremethylester;
mit 4-(4-Chlor-5-chlor-6-methyl-thieno-[2,3-d]-pyrimidin-2-yl)-benzoesäuremethylester
   4-(4-Benzylamino-5-chlor-6-methyl-thieno-[2,3-d]-pyrimidin-2-yl)-benzoesäuremethylester;
mit 4-(4-Chlor-6-nitro-thieno-[2,3-d]-pyrimidin-2-yl])benzoesäuremethylester
   4-(4-Benzylamino-6-nitro-thieno-[2,3-d]-pyrimidin-2-yl)-benzoesäuremethylester;
mit 4-(4-Chlor-5,6-dimethyl-thieno-[2,3-d]-pyrimidin-2-yl)-benzoesäuremethylester
   4-(4-Benzylamino-5,6-dimethyl-thieno-[2,3-d]-pyrimidin-2-yl)-benzoesäuremethylester;
mit 4-(4-Chlor-6-trifluormethyl-thieno-[2,3-d]-pyrimidin-2-yl)-benzoesäuremethylester
   4-(4-Benzylamino-6-trifluormethyl-thieno-[2,3-d]-pyrimidin-2-yl)-benzoesäuremethylester.

Analog erhält man durch Umsetzung von 4-Fluorbenzylamin
mit 4-(4-Chlor-6-methyl-thieno-[2,3-d]-pyrimidin-2-yl)-benzoesäuremethylester
   4-[4-(4-Fluorbenzylamino)-6-methyl-thieno-[2,3-d]-pyrimidin-2-yl]-benzoesäuremethylester;
mit 4-(4-Chlor-5-methyl-thieno-[2,3-d]-pyrimidin-2yl)-benzoesäuremethylester
   4-[4-(4-Fluorbenzylamino)-5-methyl-thieno-[2,3-d]-pyrimidin-2-yl]-benzoesäuremethylester;
mit 4-(4-Chlor-5,6,7,8-tetrahydro-[1]-benzothieno-[2,3-d]-pyrimidin-2-yl)-benzoesäuremethylester
   4-[4-(4-Fluorbenzylamino)-5,6,7,8-tetrahydro-[1]-benzothieno-[2,3-d]-pyrimidin-2-yl]-benzoesäuremethylester;
mit 4-(4-Chlor-5,6-cyclopenteno-thieno-[2,3-d]-pyrimidin-2-yl)-benzoesäuremethylester
   4-[4-(4-Fluorbenzylamino)-5,6-cyclopenteno-thieno-[2,3-d]-pyrimidin-2-yl]-benzoesäuremethylester;
mit 4-(4-Chlor-5,6-cyclohepteno-thieno-[2,3-d]-pyrimidin-2-yl)-benzoesäuremethylester
   4-[4-(4-Fluorbenzylamino)-5,6-cyclohepteno-thieno-[2,3-d]-pyrimidin-2-yl]-benzoesäuremethylester;
mit 4-(4-Chlor-6-ethyl-thieno-[2,3-d]-pyrimidin-2-yl)-benzoesäuremethylester
   4-[4-(4-Fluorbenzylamino)-6-ethyl-thieno-[2,3-d]-pyrimidin-2-yl]-benzoesäuremethylester;
mit 4-(4-Chlor-6-chlor-thieno-[2,3-d]-pyrimidin-2-yl)-benzoesäuremethylester
   4-[4-(4-Fluorbenzylamino)-6-chlor-thieno-[2,3-d]-pyrimidin-2-yl]-benzoesäuremethylester;
mit 4-(4-Chlor-5-chlor-6-methyl-thieno-[2,3-d]-pyrimidin-2-yl)-benzoesäuremethylester
   4-[4-(4-Fluorbenzylamino)-5-chlor-6-methyl-thieno-[2,3-d]-pyrimidin-2-yl]-benzoesäuremethylester;
mit 4-(4-Chlor-6-nitro-thieno-[2,3-d]-pyrimidin-2-yl)-benzoesäuremethylester
   4-[4-(4-Fluorbenzylamino)-6-nitro-thieno-[2,3-d]-pyrimidin-2-yl]-benzoesäuremethylester;
mit 4-(4-Chlor-5,6-dimethyl-thieno-[2,3-d]-pyrimidin-2-yl)-benzoesäuremethylester
   4-[4-(4-Fluorbenzylamino)-5,6-dimethyl-thieno-[2,3-d]-pyrimidin-2-yl]-benzoesäuremethylester;
mit 4-(4-Chlor-6-trifluormethyl-thieno-[2,3-d]-pyrimidin-2-yl)-benzoesäuremethylester
   4-[4-(4-Fluorbenzylamino)-6-trifluormethyl-thieno-[2,3-d]-pyrimidin-2-yl]-benzoesäuremethylester.

Analog erhält man durch Umsetzung von 3,4-Dichlorbenzylamin
mit 4-(4-Chlor-6-methyl-thieno-[2,3-d]-pyrimidin-2-yl)-benzoesäuremethylester
   4-[4-(3,4-Dichlorbenzylamino)-6-methyl-thieno-[2,3-d]-pyrimidin-2-yl]-benzoesäuremethylester;
mit 4-(4-Chlor-5-methyl-thieno-[2,3-d]-pyrimidin-2yl)-benzoesäuremethylester
   4-[4-(3,4-Dichlorbenzylamino)-5-methyl-thieno-[2,3-d]-pyrimidin-2-yl]-benzoesäuremethylester;
mit 4-(4-Chlor-5,6,7,8-tetrahydro-[1]-benzothieno-[2,3-d]-pyrimidin-2-yl)-benzoesäuremethylester
   4-[4-(3,4-Dichlorbenzylamino)-5,6,7,8-tetrahydro-[1]-benzothieno-[2,3-d]-pyrimidin-2-yl]-benzoesäuremethylester;
mit 4-(4-Chlor-5,6-cyclopenteno-thieno-[2,3-d]-pyrimidin-2-yl)-benzoesäuremethylester
   4-[4-(3,4-Dichlorbenzylamino)-5,6-cyclopenteno-thieno-[2,3-d]-pyrimidin-2-yl]-benzoesäuremethylester;
mit 4-(4-Chlor-5,6-cyclohepteno-thieno-[2,3-d]-pyrimidin-2-yl)-benzoesäuremethylester
   4-[4-(3,4-Dichlorbenzylamino)-5,6-cyclohepteno-thieno-[2,3-d]-pyrimidin-2-yl]-benzoesäuremethylester;
mit 4-(4-Chlor-6-ethyl-thieno-[2,3-d]-pyrimidin-2-yl)-benzoesäuremethylester
   4-[4-(3,4-Dichlorbenzylamino)-6-ethyl-thieno-[2,3-d]-pyrimidin-2-yl]-benzoesäuremethylester;
mit 4-(4-Chlor-6-chlor-thieno-[2,3-d]-pyrimidin-2-yl)-benzoesäuremethylester
   4-[4-(3,4-Dichlorbenzylamino)-6-chlor-thieno-[2,3-d]-pyrimidin-2-yl]-benzoesäuremethylester;
mit 4-(4-Chlor-5-chlor-6-methyl-thieno-[2,3-d]-pyrimidin-2-yl)-benzoesäuremethylester
   4-[4-(3,4-Dichlorbenzylamino)-5-chlor-6-methyl-thieno-[2,3-d]-pyrimidin-2-yl]-benzoesäuremethylester;
mit 4-(4-Chlor-6-nitro-thieno-[2,3-d]-pyrimidin-2-yl)-benzoesäuremethylester
   4-[4-(3,4-Dichlorbenzylamino)-6-nitro-thieno-[2,3-d]-pyrimidin-2-yl]-benzoesäuremethylester;
mit 4-(4-Chlor-5,6-dimethyl-thieno-[2,3-d]-pyrimidin-2-yl)-benzoesäuremethylester
   4-[4-(3,4-Dichlorbenzylamino)-5,6-dimethyl-thieno-[2,3-d]-pyrimidin-2-yl]-benzoesäuremethylester;
mit 4-(4-Chlor-6-trifluormethyl-thieno-[2,3-d]-pyrimidin-2-yl)-benzoesäuremethylester
   4-[4-(3,4-Dichlorbenzylamino)-6-trifluormethyl-thieno-[2,3-d]-pyrimidin-2-yl]-benzoesäuremethylester.

Analog erhält man durch Umsetzung von 3-Nitrobenzylamin
mit 4-(4-Chlor-6-methyl-thieno-[2,3-d]-pyrimidin-2-yl)-benzoesäuremethylester
   4-[4-(3-Nitrobenzylamino)-6-methyl-thieno-[2,3-d]-pyrimidin-2-yl]-benzoesäuremethylester;
mit 4-(4-Chlor-5-methyl-thieno-[2,3-d]-pyrimidin-2yl)-benzoesäuremethylester
   4-[4-(3-Nitrobenzylamino)-5-methyl-thieno-[2,3-d]-pyrimidin-2-yl]-benzoesäuremethylester;
mit 4-(4-Chlor-5,6,7,8-tetrahydro-[1]-benzothieno-[2,3-d]-pyrimidin-2-yl)-benzoesäuremethylester
   4-[4-(3-Nitrobenzylamino)-5,6,7,8-tetrahydro-[1]-benzothieno-[2,3-d]-pyrimidin-2-yl]-benzoesäuremethylester;
mit 4-(4-Chlor-5,6-cyclopenteno-thieno-[2,3-d]-pyrimidin-2-yl)-benzoesäuremethylester
   4-[4-(3-Nitrobenzylamino)-5,6-cyclopenteno-thieno-[2,3-d]-pyrimidin-2-yl]-benzoesäuremethylester;
mit 4-(4-Chlor-5,6-cyclohepteno-thieno-[2,3-d]-pyrimidin-2-yl)-benzoesäuremethylester
   4-[4-(3-Nitrobenzylamino)-5,6-cyclohepteno-thieno-[2,3-d]-pyrimidin-2-yl]-benzoesäuremethylester;
mit 4-(4-Chlor-6-ethyl-thieno-[2,3-d]-pyrimidin-2-yl)-benzoesäuremethylester
   4-[4-(3-Nitrobenzylamino)-6-ethyl-thieno-[2,3-d]-pyrimidin-2-yl]-benzoesäuremethylester;
mit 4-(4-Chlor-6-chlor-thieno-[2,3-d]-pyrimidin-2-yl)-benzoesäuremethylester
   4-[4-(3-Nitrobenzylamino)-6-chlor-thieno-[2,3-d]-pyrimidin-2-yl]-benzoesäuremethylester;
mit 4-(4-Chlor-5-chlor-6-methyl-thieno-[2,3-d]-pyrimidin-2-yl)-benzoesäuremethylester
   4-[4-(3-Nitrobenzylamino)-5-chlor-6-methyl-thieno-[2,3-d]-pyrimidin-2-yl]-benzoesäuremethylester;
mit 4-(4-Chlor-6-nitro-thieno-[2,3-d]-pyrimidin-2-yl)-benzoesäuremethylester
   4-[4-(3-Nitrobenzylamino)-6-nitro-thieno-[2,3-d]-pyrimidin-2-yl]-benzoesäuremethylester;
mit 4-(4-Chlor-5,6-dimethyl-thieno-[2,3-d]-pyrimidin-2-yl)-benzoesäuremethylester
   4-[4-(3-Nitrobenzylamino)-5,6-dimethyl-thieno-[2,3-d]-pyrimidin-2-yl]-benzoesäuremethylester;
mit 4-(4-Chlor-6-trifluormethyl-thieno-[2,3-d]-pyrimidin-2-yl)-benzoesäuremethylester
   4-[4-(3-Nitrobenzylamino)-6-trifluormethyl-thieno-[2,3-d]-pyrimidin-2-yl]-benzoesäuremethylester.

Analog erhält man durch Umsetzung von 3,4-Methylendioxyphenethylamin
mit 4-(4-Chlor-6-methyl-thieno-[2,3-d]-pyrimidin-2-yl)-benzoesäuremethylester
   4-[4-(3,4-Methylendioxyphenethylamino)-6-methyl-thieno-[2,3-d]-pyrimidin-2-yl]-benzoesäuremethylester;
mit 4-(4-Chlor-5-methyl-thieno-[2,3-d]-pyrimidin-2yl)-benzoesäuremethylester
   4-[4-(3,4-Methylendioxyphenethylamino)-5-methyl-thieno-[2,3-d]-pyrimidin-2-yl]-benzoesäuremethylester;
mit 4-(4-Chlor-5,6,7,8-tetrahydro-[1]-benzothieno-[2,3-d]-pyrimidin-2-yl)-benzoesäuremethylester
   4-[4-(3,4-Methylendioxyphenethylamino)-5,6,7,8-tetrahydro-[1]-benzothieno-[2,3-d]-pyrimidin-2-yl]-benzoesäuremethylester;
mit 4-(4-Chlor-5,6-cyclopenteno-thieno-[2,3-d)-pyrimidin-2-yl)-benzoesäuremethylester
   4-[4-(3,4-Methylendioxyphenethylamino)-5,6-cyclopenteno-thieno-[2,3-d]-pyrimidin-2-yl]-benzoesäuremethylester;
mit 4-(4-Chlor-5,6-cyclohepteno-thieno-[2,3-d]-pyrimidin-2-yl)-benzoesäuremethylester
   4-[4-(3,4-Methylendioxyphenethylamino)-5,6-cyclohepteno-thieno-[2,3-d]-pyrimidin-2-yl]-benzoesäuremethylester;
mit 4-(4-Chlor-6-ethyl-thieno-[2,3-d]-pyrimidin-2-yl)-benzoesäuremethylester
   4-[4-(3,4-Methylendioxyphenethylamino)-6-ethyl-thieno-[2,3-d]-pyrimidin-2-yl]-benzoesäuremethylester;
mit 4-(4-Chlor-6-chlor-thieno-[2,3-d]-pyrimidin-2-yl)-benzoesäuremethylester
   4-[4-(3,4-Methylendioxyphenethylamino)-6-chlor-thieno-[2,3-d]-pyrimidin-2-yl]-benzoesäuremethylester;
mit 4-(4-Chlor-5-chlor-6-methyl-thieno-[2,3-d]-pyrimidin-2-yl)-benzoesäuremethylester
   4-[4-(3,4-Methylendioxyphenethylamino)-5-chlor-6-methyl-thieno-[2,3-d]-pyrimidin-2-yl]-benzoesäuremethylester;
mit 4-(4-Chlor-6-nitro-thieno-[2,3-d]-pyrimidin-2-yl)-benzoesäuremethylester
   4-[4-(3,4-Methylendioxyphenethylamino)-6-nitro-thieno-[2,3-d]-pyrimidin-2-yl]-benzoesäuremethylester;
mit 4-(4-Chlor-5,6-dimethyl-thieno-[2,3-d]-pyrimidin-2-yl)-benzoesäuremethylester
   4-[4-(3,4-Methylendioxyphenethylamino)-5,6-dimethyl-thieno-[2,3-d]-pyrimidin-2-yl]-benzoesäuremethylester;
mit 4-(4-Chlor-6-trifluormethyl-thieno-[2,3-d]-pyrimidin-2-yl)-benzoesäuremethylester
   4-[4-(3,4-Methylendioxyphenethylamino)-6-trifluormethyl-thieno-[2,3-d]-pyrimidin-2-yl]-benzoesäuremethylester.

Analog erhält man durch Umsetzung von 3,4-Ethylendioxybenzylamin
mit 4-(4-Chlor-6-methyl-thieno-[2,3-d]-pyrimidin-2-yl)-benzoesäuremethylester
   4-[4-(3,4-Ethylendioxyphenethylamino)-6-methyl-thieno-[2,3-d]-pyrimidin-2-yl]-benzoesäuremethylester;
mit 4-(4-Chlor-5-methyl-thieno-[2,3-d]-pyrimidin-2yl)-benzoesäuremethylester
   4-[4-(3,4-Ethylendioxyphenethylamino)-5-methyl-thieno-[2,3-d]-pyrimidin-2-yl]-benzoesäuremethylester;
mit 4-(4-Chlor-5,6,7,8-tetrahydro-[1]-benzothieno-[2,3-d]-pyrimidin-2-yl)-benzoesäuremethylester
   4-[4-(3,4-Ethylendioxyphenethylamino)-5,6,7,8-tetrahydro-[1]-benzothieno-[2,3-d]-pyrimidin-2-yl]-benzoesäuremethylester;
mit 4-(4-Chlor-5,6-cyclopenteno-thieno-[2,3-d]-pyrimidin-2-yl)-benzoesäuremethylester
   4-[4-(3,4-Ethylendioxyphenethylamino)-5,6-cyclopenteno-thieno-[2,3-d]-pyrimidin-2-yl]-benzoesäuremethylester;
mit 4-(4-Chlor-5,6-cyclohepteno-thieno-[2,3-d]-pyrimidin-2-yl)-benzoesäuremethylester
   4-[4-(3,4-Ethylendioxyphenethylamino)-5,6-cyclohepteno-thieno-[2,3-d]-pyrimidin-2-yl]-benzoesäuremethylester;
mit 4-(4-Chlor-6-ethyl-thieno-[2,3-d]-pyrimidin-2-yl)-benzoesäuremethylester
   4-[4-(3,4-Ethylendioxyphenethylamino)-6-ethyl-thieno-[2,3-d]-pyrimidin-2-yl]-benzoesäuremethylester;
mit 4-(4-Chlor-6-chlor-thieno-[2,3-d]-pyrimidin-2-yl)-benzoesäuremethylester
   4-[4-(3,4-Ethylendioxyphenethylamino)-6-chlor-thieno-[2,3-d]-pyrimidin-2-yl]-benzoesäuremethylester;
mit 4-(4-Chlor-5-chlor-6-methyl-thieno-[2,3-d]-pyrimidin-2-yl)-benzoesäuremethylester
   4-[4-(3,4-Ethylendioxyphenethylamino)-5-chlor-6-methyl-thieno-[2,3-d]-pyrimidin-2-yl]-benzoesäuremethylester;
mit 4-(4-Chlor-6-nitro-thieno-[2,3-d]-pyrimidin-2-yl)-benzoesäuremethylester
   4-[4-(3,4-Ethylendioxyphenethylamino)-6-nitro-thieno-[2,3-d]-pyrimidin-2-yl]-benzoesäuremethylester;
mit 4-(4-Chlor-5,6-dimethyl-thieno-[2,3-d]-pyrimidin-2-yl)-benzoesäuremethylester
   4-[4-(3,4-Ethylendioxyphenethylamino)-5,6-dimethyl-thieno-[2,3-d]-pyrimidin-2-yl]-benzoesäuremethylester;
mit 4-(4-Chlor-6-trifluormethyl-thieno-[2,3-d]-pyrimidin-2-yl)-benzoesäuremethylester
   4-[4-(3,4-Ethylendioxyphenethylamino)-6-trifluormethyl-thieno-[2,3-d]-pyrimidin-2-yl]-benzoesäuremethylester.

Analog erhält man durch Umsetzung von Phenethylamin
mit 4-(4-Chlor-6-methyl-thieno-[2,3-d]-pyrimidin-2-yl)-benzoesäuremethylester
   4-(4-Phenethylamino-6-methyl-thieno-[2,3-d]-pyrimidin-2-yl)-benzoesäuremethylester;
mit 4-(4-Chlor-5-methyl-thieno-[2,3-d]-pyrimidin-2yl)-benzoesäuremethylester
   4-(4-Phenethylamino-5-methyl-thieno-[2,3-d]-pyrimidin-2-yl)-benzoesäuremethylester;
mit 4-(4-Chlor-5,6,7,8-tetrahydro-[1]-benzothieno-[2,3-d]-pyrimidin-2-yl)-benzoesäuremethylester
   4-(4-Phenethylamino-5,6,7,8-tetrahydro-[1]-benzothieno-[2,3-d]-pyrimidin-2-yl)-benzoesäuremethylester;
mit 4-(4-Chlor-5,6-cyclopenteno-thieno-[2,3-d]-pyrimidin-2-yl)-benzoesäuremethylester
   4-(4-Phenethylamino-5,6-cyclopenteno-thieno-[2,3-d]-pyrimidin-2-yl)-benzoesäuremethylester;
mit 4-(4-Chlor-5,6-cyclohepteno-thieno-[2,3-d]-pyrimidin-2-yl)-benzoesäuremethylester
   4-(4-Phenethylamino-5,6-cyclohepteno-thieno-[2,3-d]-pyrimidin-2-yl)-benzoesäuremethylester;
mit 4-(4-Chlor-6-ethyl-thieno-[2,3-d]-pyrimidin-2-yl)-benzoesäuremethylester
   4-(4-Phenethylamino-6-ethyl-thieno-[2,3-d]-pyrimidin-2-yl)-benzoesäuremethylester;
mit 4-(4-Chlor-6-chlor-thieno-[2,3-d]-pyrimidin-2-yl)-benzoesäuremethylester
   4-(4-Phenethylamino-6-chlor-thieno-[2,3-d]-pyrimidin-2-yl)-benzoesäuremethylester;
mit 4-(4-Chlor-5-chlor-6-methyl-thieno-[2,3-d]-pyrimidin-2-yl)-benzoesäuremethylester
   4-(4-Phenethylamino-5-chlor-6-methyl-thieno-[2,3-d]-pyrimidin-2-yl)-benzoesäuremethylester;
mit 4-(4-Chlor-6-nitro-thieno-[2,3-d]-pyrimidin-2-yl)-benzoesäuremethylester
   4-(4-Phenethylamino-6-nitro-thieno-[2,3-d]-pyrimidin-2-yl)-benzoesäuremethylester;
mit 4-(4-Chlor-5,6-dimethyl-thieno-[2,3-d]-pyrimidin-2-yl)-benzoesäuremethylester
   4-(4-Phenethylamino-5,6-dimethyl-thieno-[2,3-d]-pyrimidin-2-yl)-benzoesäuremethylester;
mit 4-(4-Chlor-6-trifluormethyl-thieno-[2,3-d]-pyrimidin-2-yl)-benzoesäuremethylester
   4-(4-Phenethylamino-6-trifluormethyl-thieno-[2,3-d]-pyrimidin-2-yl)-benzoesäuremethytester.

### Beispiel 6

Eine Lösung aus 1,1 g 4-[4-(3,4-Methylendioxybenzylamino)-6-methylthieno-[2,3-d]-pyrimidin-2-yl]-benzoesäuremethylester, 30 ml 2N NaOH und 30 ml Tetrahydrofuran wird 6 Stunden auf 100° erwärmt. Nach Abkühlen und Ansäuern der Lösung mit 20 %iger HCI wird wie üblich weiter aufgearbeitet. Man erhält 0,75 g 4-[4-(3,4-Methylendioxybenzylamino)-6-methyl-thieno-[2,3-d]-pyrimidin-2-yl]-benzoesäure, F. >250°.

Analog erhält man aus den in Beispiel 5 erhaltenen Estern die nachstehenden Carbonsäuren
4-[4-(3,4-Methylendioxybenzyl)-5-methyl-thieno-[2,3-d]-pyrimidin-2-yl]-benzoesäure;
4-[4-(3,4-Methylendioxybenzylamino)-5,6,7,8-tetrahydro-[1]-benzothieno-[2,3-d]-pyrimidin-2-yl]-benzoesäure, Dihydrat, F. 249°; Natriumsalz, F. >250°;
4-[4-(3,4-Methylendioxybenzylamino)-5,6-cyclopenteno-thieno-[2,3-d]-pyrimidin-2-yl]-benzoesäure;
4-[4-(3,4-Methylendioxybenzylamino)-5,6-cyclohepteno-thieno-[2,3-d]-pyrimidin-2-yl]-benzoesäure;
4-[4-(3,4-Methylendioxybenzylamino)-6-ethyl-thieno-[2,3-d]-pyrimidin-2-yl]-benzoesäure, F. 189°;
4-[4-(3,4-Methylendioxybenzylamino)-6-propyl-thieno-[2,3-d]-pyrimidin-2-yl]-benzoesäure;
4-[4-(3,4-Methylendioxybenzylamino)-6-chlor-thieno-[2,3-d]-pyrimidin-2-yl]-benzoesäure, F. >250°;
4-[4-(3,4-Methylendioxybenzylamino)-5-chlor-6-methyl-thieno-[2,3-d]-pyrimidin-2-yl]-benzoesäure, F. >250°;
4-[4-(3,4-Methylendioxybenzylamino)-6-nitro-thieno-[2,3-d]-pyrimidin-2-yl]-benzoesäure;
4-[4-(3,4-Methylendioxybenzylamino)-5,6-dimethyl-thieno-[2,3-d]-pyrimidin-2-yl]-benzoesäure, F. 172°;
4-[4-(3,4-Methylendioxybenzylamino)-6-trifluormethyl-thieno-[2,3-d]-pyrimidin-2-yl]-benzoesäure;
4-[4-(3-Chlor-4-methoxybenzylamino)-6-methyl-thieno-[2,3-d]-pyrimidin-2-yl]-benzoesäure;
4-[4-(3-Chlor-4-methoxybenzylamino)-5-methyl-thieno-[2,3-d]-pyrimidin-2-yl]-benzoesäure;
4-[4-(3-Chlor-4-methoxybenzylamino)-5,6,7,8-tetrahydro-[1]-benzothieno-[2,3-d]-pyrimidin-2-yl]-benzoesäure, F. 245°;
4-[4-(3-Chlor-4-methoxybenzylamino)-5,6-cyclopenteno-thieno-[2,3-d]-pyrimidin-2-yl]-benzoesäure;
4-[4-(3-Chlor-4-methoxybenzylamino)-5,6-cyclohepteno-thieno-[2,3-d]-pyrimidin-2-yl]-benzoesäure;
4-[4-(3-Chlor-4-methoxybenzylamino)-6-ethyl-thieno-[2,3-d]-pyrimidin-2-yl]-benzoesäure, F. 257°;
4-[4-(3-Chlor-4-methoxybenzylamino)-6-chlor-thieno-[2,3-d]-pyrimidin-2-yl]-benzoesäure, F. >250°;
4-[4-(3-Chlor-4-methoxybenzylamino)-5-chlor-6-methyl-thieno-[2,3-d]-pyrimidin-2-yl]-benzoesäure, Natriumsalz, F. >250°;
4-[4-(3-Chlor-4-methoxybenzylamino)-6-nitro-thieno-[2,3-d]-pyrimidin-2-yl]-benzoesäure;
4-[4-(3-Chlor-4-methoxybenzylamino)-5,6-dimethyl-thieno-[2,3-d]-pyrimidin-2-yl]-benzoesäure;
4-[4-(3-Chlor-4-methoxybenzylamino)-6-trifluormethyl-thieno-[2,3-d]-pyrimidin-2-yl]-benzoesäure;
4-[4-(3,4-Dimethoxybenzylamino)-6-methyl-thieno-[2,3-d]-pyrimidin-2-yl]-benzoesäure;
4-[4-(3,4-Dimethoxybenzylamino)-5-methyl-thieno-[2,3-d]-pyrimidin-2-yl]-benzoesäure;
4-[4-(3,4-Dimethoxybenzylamino)-5,6,7,8-tetrahydro-[1]-benzothieno-[2,3-d]-pyrimidin-2-yl]-benzoesäure;
4-[4-(3,4-Dimethoxybenzylamino)-5,6-cyclopenteno-thieno-[2,3-d]-pyrimidin-2-yl]-benzoesäure;
4-[4-(3,4-Dimethoxybenzylamino)-5,6-cyclohepteno-thieno-[2,3-d]-pyrimidin-2-yl]-benzoesäure;
4-[4-(3,4-Dimethoxybenzylamino)-6-ethyl-thieno-[2,3-d]-pyrimidin-2-yl]-benzoesäure;
4-[4-(3,4-Dimethoxybenzylamino)-6-chlor-thieno-[2,3-d]-pyrimidin-2-yl]-benzoesäure;
4-[4-(3,4-Dimethoxybenzylamino)-5-chlor-6-methyl-thieno-[2,3-d]-pyrimidin-2-yl]-benzoesäure;
4-[4-(3,4-Dimethoxybenzylamino)-6-nitro-thieno-[2,3-d]-pyrimidin-2-yl]-benzoesäure;
4-[4-(3,4-Dimethoxybenzylamino)-5,6-dimethyl-thieno-[2,3-d]-pyrimidin-2-yl]-benzoesäure;
4-[4-(3,4-Dimethoxybenzylamino)-6-trifluormethyl-thieno-[2,3-d]-pyrimidin-2-yl]-benzoesäure;
4-(4-Benzylamino-6-methyl-thieno-[2,3-d]-pyrimidin-2-yl)-benzoesäure, F. >250°;
4-(4-Benzylamino-5-methyl-thieno-[2,3-d]-pyrimidin-2-yl)-benzoesäure;
4-(4-Benzylamino-5,6,7,8-tetrahydro-[1]-benzothieno-[2,3-d]-pyrimidin-2-yl)-benzoesäure, F. > 270°;
4-(4-Benzylamino-5,6-cyclopenteno-thieno-[2,3-d]-pyrimidin-2-yl)-benzoesäure;
4-(4-Benzylamino)-5,6-cyclohepteno-thieno-[2,3-d]-pyrimidin-2-yl)-benzoesäure;
4-(4-Benzylamino-6-ethyl-thieno-[2,3-d]-pyrimidin-2-yl)-benzoesäure, F. 172°;
4-(4-Benzylamino-6-chlor-thieno-[2,3-d]-pyrimidin-2-yl)-benzoesäure;
4-(4-Benzylamino-5-chlor-6-methyl-thieno-[2,3-d]-pyrimidin-2-yl)-benzoesäure;
4-(4-Benzylamino-6-nitro-thieno-[2,3-d]-pyrimidin-2-yl)-benzoesäure;
4-(4-Benzylamino-5,6-dimethyl-thieno-[2,3-d]-pyrimidin-2-yl)-benzoesäure;
4-(4-Benzylamino-6-trifluormethyl-thieno-[2,3-d]-pyrimidin-2-yl)-benzoesäure;
4-[4-(4-Fluorbenzylamino)-6-methyl-thieno-[2,3-d]-pyrimidin-2-yl]-benzoesäure;
4-[4-(4-Fluorbenzylamino)-5-methyl-thieno-[2,3-d]-pyrimidin-2-yl]-benzoesäure;
4-[4-(4-Fluorbenzylamino)-5,6,7,8-tetrahydro-[1]-benzothieno-[2,3-d]-pyrimidin-2-yl]-benzoesäure;
4-[4-(4-Fluorbenzylamino)-5,6-cyclopenteno-thieno-[2,3-d]-pyrimidin-2-yl]-benzoesäure;
4-[4-(4-Fluorbenzylamino)-5,6-cyclohepteno-thieno-[2,3-d]-pyrimidin-2-yl]-benzoesäure;
4-[4-(4-Fluorbenzylamino)-6-ethyl-thieno-[2,3-d]-pyrimidin-2-yl]-benzoesäure;
4-[4-(4-Fluorbenzylamino)-6-chlor-thieno-[2,3-d]-pyrimidin-2-yl]-benzoesäure;
4-[4-(4-Fluorbenzylamino)-5-chlor-6-methyl-thieno-[2,3-d]-pyrimidin-2-yl]-benzoesäure;
4-[4-(4-Fluorbenzylamino)-6-nitro-thieno-[2,3-d]-pyrimidin-2-yl]-benzoesäure;
4-[4-(4-Fluorbenzylamino)-5,6-dimethyl-thieno-[2,3-d]-pyrimidin-2-yl]-benzoesäure;
4-[4-(4-Fluorbenzylamino)-6-trifluormethyl-thieno-[2,3-d]-pyrimidin-2-yl]-benzoesäure;
4-[4-(3,4-Dichlorbenzylamino)-6-methyl-thieno-[2,3-d]-pyrimidin-2-yl]-benzoesäure;
4-[4-(3,4-Dichlorbenzylamino)-5-methyl-thieno-[2,3-d]-pyrimidin-2-yl]-benzoesäure;
4-[4-(3,4-Dichlorbenzylamino)-5,6,7,8-tetrahydro-[1]-benzothieno-[2,3-d]-pyrimidin-2-yl]-benzoesäure;
4-[4-(3,4-Dichlorbenzylamino)-5,6-cyclopenteno-thieno-[2,3-d]-pyrimidin-2-yl]-benzoesäure;
4-[4-(3,4-Dichlorbenzylamino)-5,6-cyclohepteno-thieno-[2,3-d]-pyrimidin-2-yl]-benzoesäure;
4-[4-(3,4-Dichlorbenzylamino)-6-ethyl-thieno-[2,3-d]-pyrimidin-2-yl]-benzoesäure;
4-[4-(3,4-Dichlorbenzylamino)-6-chlor-thieno-[2,3-d]-pyrimidin-2-yl]-benzoesäure;
4-[4-(3,4-Dichlorbenzylamino)-5-chlor-6-methyl-thieno-[2,3-d]-pyrimidin-2-yl]-benzoesäure;
4-[4-(3,4-Dichlorbenzylamino)-6-nitro-thieno-[2,3-d]-pyrimidin-2-yl]-benzoesäure;
4-[4-(3,4-Dichlorbenzylamino)-5,6-dimethyl-thieno-[2,3-d]-pyrimidin-2-yl]-benzoesäure;
4-[4-(3,4-Dichlorbenzylamino)-6-trifluormethyl-thieno-[2,3-d]-pyrimidin-2-yl]-benzoesäure;
4-[4-(3-Nitrobenzylamino)-6-methyl-thieno-[2,3-d]-pyrimidin-2-yl]-benzoesäure;
4-[4-(3-Nitrobenzylamino)-5-methyl-thieno-[2,3-d]-pyrimidin-2-yl]-benzoesäure;
4-[4-(3-Nitrobenzylamino)-5,6,7,8-tetrahydro-[1]-benzothieno-[2,3-d]-pyrimidin-2-yl]-benzoesäure;
4-[4-(3-Nitrobenzylamino)-5,6-cyclopenteno-thieno-[2,3-d]-pyrimidin-2-yl]-benzoesäure;
4-[4-(3-Nitrobenzylamino)-5,6-cyclohepteno-thieno-[2,3-d]-pyrimidin-2-yl]-benzoesäure;
4-[4-(3-Nitrobenzylamino)-6-ethyl-thieno-[2,3-d]-pyrimidin-2-yl]-benzoesäure;
4-[4-(3-Nitrobenzylamino)-6-chlor-thieno-[2,3-d]-pyrimidin-2-yl]-benzoesäure;
4-[4-(3-Nitrobenzylamino)-5-chlor-6-methyl-thieno-[2,3-d]-pyrimidin-2-yl]-benzoesäure;
4-[4-(3-Nitrobenzylamino)-6-nitro-thieno-[2,3-d]-pyrimidin-2-yl]-benzoesäure;
4-[4-(3-Nitrobenzylamino)-5,6-dimethyl-thieno-[2,3-d]-pyrimidin-2-yl]-benzoesäure;
4-[4-(3-Nitrobenzylamino)-6-trifluormethyl-thieno-[2,3-d]-pyrimidin-2-yl]-benzoesäure;
4-[4-(3,4-Methylendioxyphenethylamino)-6-methyl-thieno-[2,3-d]-pyrimidin-2-yl]-benzoesäure;
4-[4-(3,4-Methylendioxyphenethylamino)-5-methyl-thieno-[2,3-d]-pyrimidin-2-yl]-benzoesäure;
4-[4-(3,4-Methylendioxyphenethylamino)-5,6,7,8-tetrahydro-[1]-benzothieno-[2,3-d]-pyrimidin-2-yl]-benzoesäure;
4-[4-(3,4-Methylendioxyphenethylamino)-5,6-cyclopenteno-thieno-[2,3-d]-pyrimidin-2-yl]-benzoesäure;
4-[4-(3,4-Methylendioxyphenethylamino)-5,6-cyclohepteno-thieno-[2,3-d]-pyrimidin-2-yl]-benzoesäure;
4-[4-(3,4-Methylendioxyphenethylamino)-6-ethyl-thieno-[2,3-d]-pyrimidin-2-yl]-benzoesäure;
4-[4-(3,4-Methylendioxyphenethylamino)-6-chlor-thieno-[2,3-d]-pyrimidin-2-yl]-benzoesäure;
4-[4-(3,4-Methylendioxyphenethylamino)-5-chlor-6-methyl-thieno-[2,3-d]-pyrimidin-2-yl]-benzoesäure;
4-[4-(3,4-Methylendioxyphenethylamino)-6-nitro-thieno-[2,3-d]-pyrimidin-2-yl]-benzoesäure;
4-[4-(3,4-Methylendioxyphenethylamino)-5,6-dimethyl-thieno-[2,3-d]-pyrimidin-2-yl]-benzoesäure;
4-[4-(3,4-Methylendioxyphenethylamino)-6-trifluormethyl-thieno-[2,3-d]-pyrimidin-2-yl]-benzoesäure;
4-[4-(3,4-Ethylendioxyphenethylamino)-6-methyl-thieno-[2,3-d]-pyrimidin-2-yl]-benzoesäure
4-[4-(3,4-Ethylendioxyphenethylamino)-5-methyl-thieno-[2,3-d]-pyrimidin-2-yl]-benzoesäure;
4-[4-(3,4-Ethylendioxyphenethylamino)-5,6,7,8-tetrahydro-[1]-benzothieno-[2,3-d]-pyrimidin-2-yl]-benzoesäure;
4-[4-(3,4-Ethylendioxyphenethylamino)-5,6-cyclopenteno-thieno-[2,3-d]-pyrimidin-2-yl]-benzoesäure;
4-[4-(3,4-Ethylendioxyphenethylamino)-5,6-cyclohepteno-thieno-[2,3-d]-pyrimidin-2-yl]-benzoesäure;
4-[4-(3,4-Ethylendioxyphenethylamino)-6-ethyl-thieno-[2,3-d]-pyrimidin-2-yl]-benzoesäure;
4-[4-(3,4-Ethylendioxyphenethylamino)-6-chlor-thieno-[2,3-d]-pyrimidin-2-yl]-benzoesäure;
4-[4-(3,4-Ethylendioxyphenethylamino)-5-chlor-6-methyl-thieno-[2,3-d]-pyrimidin-2-yl]-benzoesäure;
4-[4-(3,4-Ethylendioxyphenethylamino)-6-nitro-thieno-[2,3-d]-pyrimidin-2-yl]-benzoesäure;
4-[4-(3,4-Ethylendioxyphenethylamino)-5,6-dimethyl-thieno-[2,3-d]-pyrimidin-2-yl]-benzoesäure;
4-[4-(3,4-Ethylendioxyphenethylamino)-6-trifluormethyl-thieno-[2,3-d]-pyrimidin-2-yl]-benzoesäure;
4-(4-Phenethylamino-6-methyl-thieno-[2,3-d]-pyrimidin-2-yl)-benzoesäure;
4-(4-Phenethylamino-5-methyl-thieno-[2,3-d]-pyrimidin-2-yl)-benzoesäure;
4-(4-Phenethylamino-5,6,7,8-tetrahydro-[1]-benzothieno-[2,3-d]-pyrimidin-2-yl)-benzoesäure;
4-(4-Phenethylamino-5,6-cyclopenteno-thieno-[2,3-d]-pyrimidin-2-yl)-benzoesäure;
4-(4-Phenethylamino-5,6-cyclohepteno-thieno-[2,3-d]-pyrimidin-2-yl)-benzoesäure;
4-(4-Phenethylamino-6-ethyl-thieno-[2,3-d]-pyrimidin-2-yl)-benzoesäure;
4-(4-Phenethylamino-6-chlor-thieno-[2,3-d]-pyrimidin-2-yl)-benzoesäure;
4-(4-Phenethylamino-5-chlor-6-methyl-thieno-[2,3-d]-pyrimidin-2-yl)-benzoesäure;
4-(4-Phenethylamino-6-nitro-thieno-[2,3-d]-pyrimidin-2-yl)-benzoesäure;
4-(4-Phenethylamino-5,6-dimethyl-thieno-[2,3-d]-pyrimidin-2-yl)-benzoesäure;
4-(4-Phenethylamino-6-trifluormethyl-thieno-[2,3-d]-pyrimidin-2-yl)-benzoesäure.

Analog Beispiel 5 erhält man unter Verwendung von 3-Cyanbenzoesäuremethylester und anschließender Hydrolyse die Verbindung
3-[4-(3,4-Methylendioxybenzylamino)-5,6,7,8-tetrahydro-[1]-benzothieno-[2,3-d]-pyrimidin-2-yl]-benzoesäure.

### Beispiel 7

Analog Beispiel 5 und 6 erhält man unter Verwendung der entsprechenden 4-Cyancylohexancarbonsäureester die nachstehenden Carbonsäuren
4-[4-(3,4-Methylendioxybenzylamino)-6-methyl-thieno-[2,3-d]-pyrimidin-2-yl]-cyclohexancarbonsäure;
4-[4-(3,4-Methylendioxybenzyl)-5-methyl-thieno-[2,3-d]-pyrimidin-2-yl]-cyclohexancarbonsäure;
4-[4-(3,4-Methylendioxybenzylamino)-5,6,7,8-tetrahydro-[1]-benzothieno-[2,3-d]-pyrimidin-2-yl]-cyclohexancarbonsäure, amorph;
4-[4-(3,4-Methylendioxybenzylamino)-5,6-cyclopenteno-thieno-[2,3-d]-pyrimidin-2-yl]-cyclohexancarbonsäure;
4-[4-(3,4-Methylendioxybenzylamino)-5,6-cyclohepteno-thieno-[2,3-d]-pyrimidin-2-yl]-cyclohexancarbonsäure;
4-[4-(3,4-Methylendioxybenzylamino)-6-ethyl-thieno-[2,3-d]-pyrimidin-2-yl]-cyclohexancarbonsäure;
4-[4-(3,4-Methylendioxybenzylamino)-6-chlor-thieno-[2,3-d]-pyrimidin-2-yl]-cyclohexancarbonsäure;
4-[4-(3,4-Methylendioxybenzylamino)-5-chlor-6-methyl-thieno-[2,3-d]-pyrimidin-2-yl]-cyclohexancarbonsäure;
4-[4-(3,4-Methylendioxybenzylamino)-6-nitro-thieno-[2,3-d]-pyrimidin-2-yl]-cyclohexancarbonsäure;
4-[4-(3,4-Methylendioxybenzylamino)-5,6-dimethyl-thieno-[2,3-d]-pyrimidin-2-yl]-cyclohexancarbonsäure;
4-[4-(3,4-Methylendioxybenzylamino)-6-trifluormethyl-thieno-[2,3-d]-pyrimidin-2-yl]-cyclohexancarbonsäure;
4-[4-(3-Chlor-4-methoxybenzylamino)-6-methyl-thieno-[2,3-d]-pyrimidin-2-yl]-cyclohexancarbonsäure;
4-[4-(3-Chlor-4-methoxybenzylamino)-5-methyl-thieno-[2,3-d]-pyrimidin-2-yl]-cyclohexancarbonsäure;
4-[4-(3-Chlor-4-methoxybenzylamino)-5,6,7,8-tetrahydro-[1]-benzothieno-[2,3-d]-pyrimidin-2-yl]-cyclohexancarbonsäure, amorph;
4-[4-(3-Chlor-4-methoxybenzylamino)-5,6-cyclopenteno-thieno-[2,3-d]-pyrimidin-2-yl]-cyclohexancarbonsäure;
4-[4-(3-Chlor-4-methoxybenzylamino)-5,6-cyclohepteno-thieno-[2,3-d]-pyrimidin-2-yl]-cyclohexancarbonsäure;
4-[4-(3-Chlor-4-methoxybenzylamino)-6-ethyl-thieno-[2,3-d]-pyrimidin-2-yl]-cyclohexancarbonsäure;
4-[4-(3-Chlor-4-methoxybenzylamino)-6-chlor-thieno-[2,3-d]-pyrimidin-2-yl]-cyclohexancarbonsäure;
4-[4-(3-Chlor-4-methoxybenzylamino)-5-chlor-6-methyl-thieno-[2,3-d]-pyrimidin-2-yl]-cyclohexancarbonsäure;
4-[4-(3-Chlor-4-methoxybenzylamino)-6-nitro-thieno-[2,3-d]-pyrimidin-2-yl]-cyclohexancarbonsäure;
4-[4-(3-Chlor-4-methoxybenzylamino)-5,6-dimethyl-thieno-[2,3-d]-pyrimidin-2-yl]-cyclohexancarbonsäure;
4-[4-(3-Chlor-4-methoxybenzylamino)-6-trifluormethyl-thieno-[2,3-d]-pyrimidin-2-yl]-cyclohexancarbonsäure;
4-[4-(3,4-Dimethoxybenzylamino)-6-methyl-thieno-[2,3-d]-pyrimidin-2-yl]-cyclohexancarbonsäure;
4-[4-(3,4-Dimethoxybenzylamino)-5-methyl-thieno-[2,3-d]-pyrimidin-2-yl]-cyclohexancarbonsäure;
4-[4-(3,4-Dimethoxybenzylamino)-5,6,7,8-tetrahydro-[1]-benzothieno-[2,3-d]-pyrimidin-2-yl]-cyclohexancarbonsäure;
4-[4-(3,4-Dimethoxybenzylamino)-5,6-cyclopenteno-thieno-[2,3-d]-pyrimidin-2-yl]-cyclohexancarbonsäure;
4-[4-(3,4-Dimethoxybenzylamino)-5,6-cyclohepteno-thieno-[2,3-d]-pyrimidin-2-yl]-cyclohexancarbonsäure;
4-[4-(3,4-Dimethoxybenzylamino)-6-ethyl-thieno-[2,3-d]-pyrimidin-2-yl]-cyclohexancarbonsäure;
4-[4-(3,4-Dimethoxybenzylamino)-6-chlor-thieno-[2,3-d]-pyrimidin-2-yl]-cyclohexancarbonsäure;
4-[4-(3,4-Dimethoxybenzylamino)-5-chlor-6-methyl-thieno-[2,3-d]-pyrimidin-2-yl]-cyclohexancarbonsäure;
4-[4-(3,4-Dimethoxybenzylamino)-6-nitro-thieno-[2,3-d]-pyrimidin-2-yl]-cyclohexancarbonsäure;
4-[4-(3,4-Dimethoxybenzylamino)-5,6-dimethyl-thieno-[2,3-d]-pyrimidin-2-yl]-cyclohexancarbonsäure;
4-[4-(3,4-Dimethoxybenzylamino)-6-trifluormethyl-thieno-[2,3-d]-pyrimidin-2-yl]-cyclohexancarbonsäure;
4-(4-Benzylamino-6-methyl-thieno-[2,3-d]-pyrimidin-2-yl)-cyclohexancarbonsäure;
4-(4-Benzylamino-5-methyl-thieno-[2,3-d]-pyrimidin-2-yl)-cyclohexancarbonsäure;
4-(4-Benzylamino-5,6,7,8-tetrahydro-[1]-benzothieno-[2,3-d]-pyrimidin-2-yl)-cyclohexancarbonsäure, amorph;
4-(4-Benzylamino-5,6-cyclopenteno-thieno-[2,3-d]-pyrimidin-2-yl)-cyclohexancarbonsäure;
4-(4-Benzylamino)-5,6-cyclohepteno-thieno-[2,3-d]-pyrimidin-2-yl)-cyclohexancarbonsäure;
4-(4-Benzylamino-6-ethyl-thieno-[2,3-d]-pyrimidin-2-yl)-cyclohexancarbonsäure;
4-(4-Benzylamino-6-chlor-thieno-[2,3-d]-pyrimidin-2-yl)-cyclohexancarbonsäure;
4-(4-Benzylamino-5-chlor-6-methyl-thieno-[2,3-d]-pyrimidin-2-yl)-cyclohexancarbonsäure;
4-(4-Benzylamino-6-nitro-thieno-[2,3-d]-pyrimidin-2-yl)-cyclohexancarbonsäure;
4-(4-Benzylamino-5,6-dimethyl-thieno-[2,3-d]-pyrimidin-2-yl)-cyclohexancarbonsäure;
4-(4-Benzylamino-6-trifluormethyl-thieno-[2,3-d]-pyrimidin-2-yl)-cyclohexancarbonsäure;
4-[4-(4-Fluorbenzylamino)-6-methyl-thieno-[2,3-d]-pyrimidin-2-yl]- cyclohexancarbonsäure;
4-[4-(4-Fluorbenzylamino)-5-methyl-thieno-[2,3-d]-pyrimidin-2-yl]- cyclohexancarbonsäure;
4-[4-(4-Fluorbenzylamino)-5,6,7,8-tetrahydro-[1]-benzothieno-[2,3-d]-pyrimidin-2-yl]-cyclohexancarbonsäure;
4-[4-(4-Fluorbenzylamino)-5,6-cyclopenteno-thieno-[2,3-d]-pyrimidin-2-yl]-cyclohexancarbonsäure;
4-[4-(4-Fluorbenzylamino)-5,6-cyclohepteno-thieno-[2,3-d]-pyrimidin-2-yl]-cyclohexancarbonsäure;
4-[4-(4-Fluorbenzylamino)-6-ethyl-thieno-[2,3-d]-pyrimidin-2-yl]-cyclohexancarbonsäure;
4-[4-(4-Fluorbenzylamino)-6-chlor-thieno-[2,3-d]-pyrimidin-2-yl]-cyclohexancarbonsäure;
4-[4-(4-Fluorbenzylamino)-5-chlor-6-methyl-thieno-[2,3-d]-pyrimidin-2-yl]-cyclohexancarbonsäure;
4-[4-(4-Fluorbenzylamino)-6-nitro-thieno-[2,3-d]-pyrimidin-2-yl]-cyclohexancarbonsäure;
4-[4-(4-Fluorbenzylamino)-5,6-dimethyl-thieno-[2,3-d]-pyrimidin-2-yl]-cyclohexancarbonsäure;
4-[4-(4-Fluorbenzylamino)-6-trifluormethyl-thieno-[2,3-d]-pyrimidin-2-yl]-cyclohexancarbonsäure;
4-[4-(3,4-Dichlorbenzylamino)-6-methyl-thieno-[2,3-d]-pyrimidin-2-yl]-cyclohexancarbonsäure;
4-[4-(3,4-Dichlorbenzylamino)-5-methyl-thieno-[2,3-d]-pyrimidin-2-yl]-cyclohexancarbonsäure;
4-[4-(3,4-Dichlorbenzylamino)-5,6,7,8-tetrahydro-[1]-benzothieno-[2,3-d]-pyrimidin-2-yl]-cyclohexancarbonsäure;
4-[4-(3,4-Dichlorbenzylamino)-5,6-cyclopenteno-thieno-[2,3-d]-pyrimidin-2-yl]-cyclohexancarbonsäure;
4-[4-(3,4-Dichlorbenzylamino)-5,6-cyclohepteno-thieno-[2,3-d]-pyrimidin-2-yl]-cyclohexancarbonsäure;
4-[4-(3,4-Dichlorbenzylamino)-6-ethyl-thieno-[2,3-d]-pyrimidin-2-yl]-cyclohexancarbonsäure;
4-[4-(3,4-Dichlorbenzylamino)-6-chlor-thieno-[2,3-d]-pyrimidin-2-yl]-cyclohexancarbonsäure;
4-[4-(3,4-Dichlorbenzylamino)-5-chlor-6-methyl-thieno-[2,3-d]-pyrimidin-2-yl]-cyclohexancarbonsäure;
4-[4-(3,4-Dichlorbenzylamino)-6-nitro-thieno-[2,3-d]-pyrimidin-2-yl]-cyclohexancarbonsäure;
4-[4-(3,4-Dichlorbenzylamino)-5,6-dimethyl-thieno-[2,3-d]-pyrimidin-2-yl]-cyclohexancarbonsäure;
4-[4-(3,4-Dichlorbenzylamino)-6-trifluormethyl-thieno-[2,3-d]-pyrimidin-2-yl]-cyclohexancarbonsäure;
4-[4-(3-Nitrobenzylamino)-6-methyl-thieno-[2,3-d]-pyrimidin-2-yl]-cyclohexancarbonsäure;
4-[4-(3-Nitrobenzylamino)-5-methyl-thieno-[2,3-d]-pyrimidin-2-yl]-cyclohexancarbonsäure;
4-[4-(3-Nitrobenzylamino)-5,6,7,8-tetrahydro-[1]-benzothieno-[2,3-d]-pyrimidin-2-yl]-cyclohexancarbonsäure;
4-[4-(3-Nitrobenzylamino)-5,6-cyclopenteno-thieno-[2,3-d]-pyrimidin-2-yl]-cyclohexancarbonsäure;
4-[4-(3-Nitrobenzylamino)-5,6-cyclohepteno-thieno-[2,3-d]-pyrimidin-2-yl]-cyclohexancarbonsäure;
4-[4-(3-Nitrobenzylamino)-6-ethyl-thieno-[2,3-d]-pyrimidin-2-yl]-cyclohexancarbonsäure;
4-[4-(3-Nitrobenzylamino)-6-chlor-thieno-[2,3-d]-pyrimidin-2-yl]-cyclohexancarbonsäure;
4-[4-(3-Nitrobenzylamino)-5-chlor-6-methyl-thieno-[2,3-d]-pyrimidin-2-yl]-cyclohexancarbonsäure;
4-[4-(3-Nitrobenzylamino)-6-nitro-thieno-[2,3-d]-pyrimidin-2-yl]-cyclohexancarbonsäure;
4-[4-(3-Nitrobenzylamino)-5,6-dimethyl-thieno-[2,3-d]-pyrimidin-2-yl]-cyclohexancarbonsäure;
4-[4-(3-Nitrobenzylamino)-6-trifluormethyl-thieno-[2,3-d]-pyrimidin-2-yl]-cyclohexancarbonsäure;
4-[4-(3,4-Methylendioxyphenethylamino)-6-methyl-thieno-[2,3-d]-pyrimidin-2-yl]-cyclohexancarbonsäure;
4-[4-(3,4-Methylendioxyphenethylamino)-5-methyl-thieno-[2,3-d]-pyrimidin-2-yl]-cyclohexancarbonsäure;
4-[4-(3,4-Methylendioxyphenethylamino)-5,6,7,8-tetrahydro-[1]-benzothieno-[2,3-d]-pyrimidin-2-yl]-cyclohexancarbonsäure;
4-[4-(3,4-Methylendioxyphenethylamino)-5,6-cyclopenteno-thieno-[2,3-d]-pyrimidin-2-yl]-cyclohexancarbonsäure;
4-[4-(3,4-Methylendioxyphenethylamino)-5,6-cyclohepteno-thieno-[2,3-d]-pyrimidin-2-yl]-cyclohexancarbonsäure;
4-[4-(3,4-Methylendioxyphenethylamino)-6-ethyl-thieno-[2,3-d]-pyrimidin-2-yl]-cyclohexancarbonsäure;
4-[4-(3,4-Methylendioxyphenethylamino)-6-chlor-thieno-[2,3-d]-pyrimidin-2-yl]-cyclohexancarbonsäure;
4-[4-(3,4-Methylendioxyphenethylamino)-5-chlor-6-methyl-thieno-[2,3-d]-pyrimidin-2-yl]-cyclohexancarbonsäure;
4-[4-(3,4-Methylendioxyphenethylamino)-6-nitro-thieno-[2,3-d]-pyrimidin-2-yl]-cyclohexancarbonsäure;
4-[4-(3,4-Methylendioxyphenethylamino)-5,6-dimethyl-thieno-[2,3-d]-pyrimidin-2-yl]-cyclohexancarbonsäure;
4-[4-(3,4-Methylendioxyphenethylamino)-6-trifluormethyl-thieno-[2,3-d]-pyrimidin-2-yl]-cyclohexancarbonsäure;
4-[4-(3,4-Ethylendioxyphenethylamino)-6-methyl-thieno-[2,3-d]-pyrimidin-2-yl]-cyclohexancarbonsäure;
4-[4-(3,4-Ethylendioxyphenethylamino)-5-methyl-thieno-[2,3-d]-pyrimidin-2-yl]-cyclohexancarbonsäure;
4-[4-(3,4-Ethylendioxyphenethylamino)-5,6,7,8-tetrahydro-[1]-benzothieno-[2,3-d]-pyrimidin-2-yl]-cyclohexancarbonsäure;
4-[4-(3,4-Ethylendioxyphenethylamino)-5,6-cyclopenteno-thieno-[2,3-d]-pyrimidin-2-yl]-cyclohexancarbonsäure;
4-[4-(3,4-Ethylendioxyphenethylamino)-5,6-cyclohepteno-thieno-[2,3-d]-pyrimidin-2-yl]-cyclohexancarbonsäure;
4-[4-(3,4-Ethylendioxyphenethylamino)-6-ethyl-thieno-[2,3-d]-pyrimidin-2-yl]-cyclohexancarbonsäure;
4-[4-(3,4-Ethylendioxyphenethylamino)-6-chlor-thieno-[2,3-d]-pyrimidin-2-yl]-cyclohexancarbonsäure;
4-[4-(3,4-Ethylendioxyphenethylamino)-5-chlor-6-methyl-thieno-[2,3-d]-pyrimidin-2-yl]-cyclohexancarbonsäure;
4-[4-(3,4-Ethylendioxyphenethylamino)-6-nitro-thieno-[2,3-d]-pyrimidin-2-yl]-cyclohexancarbonsäure;
4-[4-(3,4-Ethylendioxyphenethylamino)-5,6-dimethyl-thieno-[2,3-d]-pyrimidin-2-yl]-cyclohexancarbonsäure;
4-[4-(3,4-Ethylendioxyphenethylamino)-6-trifluormethyl-thieno-[2,3-d]-pyrimidin-2-yl]- cyclohexancarbonsäure;
4-(4-Phenethylamino-6-methyl-thieno-[2,3-d]-pyrimidin-2-yl)-cyclohexancarbonsäure;
4-(4-Phenethylamino-5-methyl-thieno-[2,3-d]-pyrimidin-2-yl)-cyclohexancarbonsäure;
4-(4-Phenethylamino-5,6,7,8-tetrahydro-[1]-benzothieno-[2,3-d]-pyrimidin-2-yl)-cyclohexancarbonsäure;
4-(4-Phenethylamino-5,6-cyclopenteno-thieno-[2,3-d]-pyrimidin-2-yl)-cyclohexancarbonsäure;
4-(4-Phenethylamino-5,6-cyclohepteno-thieno-[2,3-d]-pyrimidin-2-yl)-cyclohexancarbonsäure;
4-(4-Phenethylamino-6-ethyl-thieno-[2,3-d]-pyrimidin-2-yl)-cyclohexancarbonsäure;
4-(4-Phenethylamino-6-chlor-thieno-[2,3-d]-pyrimidin-2-yl)-cyclohexancarbonsäure;
4-(4-Phenethylamino-5-chlor-6-methyl-thieno-[2,3-d]-pyrimidin-2-yl)-cyclohexancarbonsäure;
4-(4-Phenethylamino-6-nitro-thieno-[2,3-d]-pyrimidin-2-yl)-cyclohexancarbonsäure;
4-(4-Phenethylamino-5,6-dimethyl-thieno-[2,3-d]-pyrimidin-2-yl)-cyclohexancarbonsäure;
4-(4-Phenethylamino-6-trifluormethyl-thieno-[2,3-d]-pyrimidin-2-yl)-cyclohexancarbonsäure.

### Beispiel 8

Eine Lösung von 4-[4-(3-Nitrobenzylamino)-5-methyl-thieno-[2,3-d]-pyrimidin-2-yl]-benzoesäure in Methanol wird in Gegenwart von Raney-Nickel hydriert. Der Katalysator wird abfiltriert und die Lösung eingeengt. Man erhält nach Umkristallisation 4-[4-(3-Aminobenzylamino)-5-methylthieno-[2,3-d]-pyrimidin-2-yl]-benzoesäure.

### Beispiel 9

Eine Lösung von 6 g 4-[4-(3-Aminobenzylamino)-5-methyl-thieno-[2,3-d]-pyrimidin-2-yl]-benzoesäure und 0,5 g Titantetrachlorid in 100 ml Methanol wird mit 1 ml frisch destilliertem Acetaldehyd versetzt. Anschließend gibt man 4 g Natriumcyanborhydrid dazu und rührt 30 Stunden. Man gibt halbkonzentrierte Salzsäure dazu, arbeitet wie üblich auf und erhält 4-[4-(3-N-Ethylaminobenzylamino)-5-methyl-thieno-[2,3-d]-pyrimidin-2-yl]-benzoesäure.

### Beispiel 10

Analog Beispiel 2 erhält man durch Umsetzung von 2-Chlor-5,6,7,8-tetrahydro-4-(3,4-methylendioxybenzylamino)-[1]-benzothieno-[2,3-d]-pyrimidin
mit Piperazin-1-yl-essigsäureethylester
   {4-[4-(3,4-Methylendioxybenzylamino)-5,6,7,8-tetrahydro-[1]-benzothieno-[2,3-d]-pyrimidin-2-yl]-piperazin-1-yl}-essigsäureethylester und
mit Piperidin-4-yl-essigsäureethylester
   {1-[4-(3,4-Methylendioxybenzylamino)-5,6,7,8-tetrahydro-[1]-benzothieno-[2,3-d]-pyrimidin-2-yl]-piperidin-4-yl}-essigsäureethylester.

Durch Esterhydrolyse erhält man daraus
{4-[4-(3,4-Methylendioxybenzylamino)-5,6,7,8-tetrahydro-[1]-benzothieno-[2,3-d]-pyrimidin-2-yl]-piperazin-1-yl}-essigsäure, F. 250° (Zers.) und
{1-[4-(3,4-Methylendioxybenzylamino)-5,6,7,8-tetrahydro-[1]- benzothieno-[2,3-d]-pyrimidin-2-yl]-piperidin-4-yl}-essigsäure, amorph.

### Beispiel 11

Analog den Beispielen 4 und 5 erhält man die nachstehenden Verbindungen
{4-[4-(3,4-Methylendioxybenzylamino)-5,6,7,8-tetrahydro-[1]- benzothieno-[2,3-d]-pyrimidin-2-yl]-phenyl}-essigsäureethylester und
{4-[4-(3-Chlor-4-methoxybenzylamino)-5,6,7,8-tetrahydro-[1]-benzothieno-[2,3-d]-pyrimidin-2-yl]-phenyl}-essigsäureethylester.

Durch Esterhydrolyse erhält man daraus
{4-[4-(3,4-Methylendioxybenzylamino)-5,6,7,8-tetrahydro-[1]- benzothieno-[2,3-d]-pyrimidin-2-yl]-phenyl}-essigsäure, F. 214° und
{4-[4-(3-Chlor-4-methoxybenzylamino)-5,6,7,8-tetrahydro-[1]-benzothieno-[2,3-d]-pyrimidin-2-yl]-phenyl}-essigsäure, Natriumsalz, F. >250°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 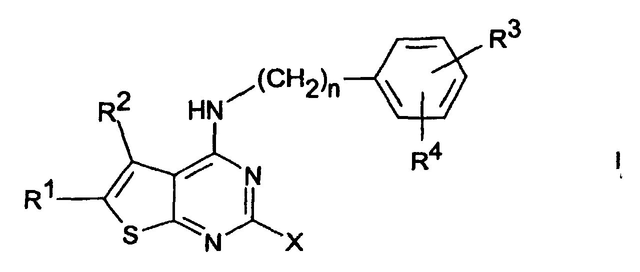 worin
R¹, R² jeweils unabhängig voneinander H, A, OA, Alkenyl, Alkinyl, CF₃ oder Hal,
wobei einer der Reste R¹ oder R² immer ≠ H ist,
R¹ und R² zusammen auch Alkylen mit 3-5 C-Atomen,
R³, R⁴ jeweils unabhängig voneinander H, A, OA, NO₂, NH₂, NHA, NAA' oder Hal,
R³ und R⁴ zusammen auch -O-CH₂-CH₂-, -O-CH₂-O- oder -O-CH₂-CH₂-O-,
X ein- oder zweifach durch COOH, CH₂COOH, COOCH₃, COOC₂H₅, CONH₂, CON(CH₃)₂, CONHCH₃ oder CN substituiertes Phenyl, Cyclopentyl, Cyclohexyl, Cycloheptyl, 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro -2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl,
A, A' jeweils unabhängig voneinander H oder Alkyl mit 1 bis 6 C-Atomen,
Hal F, Cl, Br oder I
und
n 0, 1, 2 oder 3
bedeuten,
sowie deren physiologisch unbedenklichen Salze.

2. Verbindungen der Formel I gemäß Anspruch 1
(a) 4-[4-(3,4-Methylendioxy-benzylamino)-5,6,7,8-tetrahydro-[1]-benzothieno-[2,3-d]-pyrimidin-2-yl]-benzoesäure;
(b) 4-[4-(3,4-Methylendioxy-benzylamino)-6-methyl-thieno-[2,3-d]-pyrimidin-2-yl]-benzoesäure;
(c) 4-[4-(3,4-Methylendioxy-benzylamino)-5,6-dimethyl-thieno-[2,3-d]-pyrimidin-2-yl]-benzoesäure;
(d) 4-[4-(3,4-Methylendioxy-benzylamino)-6-chlor-thieno-[2,3-d]-pyrimidin-2-yl]-benzoesäure;
(e) 4-[4-(3-Chlor-4-methoxy-benzylamino)-5,6,7,8-tetrahydro-[1]-benzothieno-[2,3-d]-pyrimidin-2-yl]-benzoesäure;
(f) 1-[4-(3,4-Methylendioxy-benzylamino)-5,8,7,8-tetrahydro-[1]-benzothieno-[2,3-d]-pyrimidin-2-yl]-piperidin-4-carbonsäure;
(g) 1-[4-(3,4-Methylendioxy-benzylamino)-6-methyl-thieno-[2,3-d]-pyrimidin-2-yl]-piperidin-4-carbonsäure;
(h) 4-[4-(3,4-Methylendioxy-benzylamino)-5,6,7,8-tetrahydro-[1]-benzothieno-[2,3-d]-pyrimidin-2-yl]-cyclohexancarbonsäure;
sowie deren physiologisch unbedenklichen Salze.

3. Verfahren zur Herstellung von Verbindungen der Formel I nach Anspruch 1 sowie deren Salzen,**dadurch gekennzeichnet,**
**daß** man eine saure Verbindung der Formel I durch Behandeln mit einer Base in eines ihrer Salze überführt.

4. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Krankheiten des Herz-Kreislaufsystems und zur Behandlung und/oder Therapie von Potenzstörungen.

7. Arzneimittel der Formel I nach Anspruch 1 und ihre physiologisch unbedenklichen Salze als Phosphodiesterase V-Hemmer.

8. Verwendung von Verbindungen der Formel I nach Anspruch 1 und/oder ihre physiologisch unbedenklichen Salze zur Herstellung eines Arzneimittels.

9. Verwendung von Verbindungen der Formel I nach Anspruch 1 und/oder ihrer physiologisch unbedenklichen Salze zur Herstellung eines Arzneimittels bei der Bekämpfung von Krankheiten.

10. Verwendung von Verbindungen der Formel I nach Anspruch 1 und/oder ihrer physiologisch unbedenklichen Salze zur Herstellung eines Arzneimittels zur Behandlung von Erkrankungen des Herz-Kreislaufsystems, insbesondere der Herzinsuffizienz und zur Behandlung und/oder Therapie von Potenzstörungen (erektile Dysfunktion).

## Claims

1. Compounds of the formula I 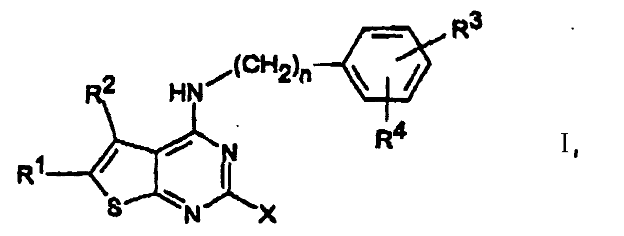 in which
R¹, R² are each, independently of one another, H, A, OA, alkenyl, alkynyl, CF₃ or Hal,
where one of the radicals R¹ and R² is always ≠ H,
R¹ and R² together are alternatively alkylene having 3-5 carbon atoms,
R³, R⁴ are each, independently of one another, H, A, OA, NO₂, NH₂, NHA, NAA' or Hal,
R³ and R⁴ together are alternatively -O-CH₂-CH₂-, -O-CH₂-O- or -O-CH₂-CH₂-O-,
X is phenyl, cyclopentyl, cyclohexyl, cycloheptyl, 2,3-dihydro-2-, -3-, -4- or -5-furyl, 2,5-dihydro-2-, -3-, -4- or -5-furyl, tetrahydro-2- or -3-furyl, 1,3-dioxolan-4-yl, tetrahydro-2- or -3-thienyl, 2,3-dihydro-1-, -2-, -3-, -4- or -5-pyrrolyl, 2,5-dihydro-1-, -2-, -3-, -4- or -5-pyrrolyl, 1-, 2- or 3-pyrrolidinyl, tetrahydro-1-, -2- or -4-imidazolyl, 2,3-dihydro-1-, -2-, -3-, -4- or -5-pyrazolyl, tetrahydro-1-, -3- or -4-pyrazolyl, 1,4-dihydro-1-, -2-, -3- or -4-pyridyl, 1,2,3,4-tetrahydro-1-, -2-, -3-, -4-, -5- or -6-pyridyl, 1-, 2-, 3- or 4-piperidinyl, 2-, 3- or 4-morpholinyl, tetrahydro-2-, -3- or -4-pyranyl, 1,4-dioxanyl, 1,3-dioxan-2-, -4- or -5-yl, hexahydro-1-, -3- or -4-pyridazinyl, hexahydro-1-, -2-, -4- or -5-pyrimidinyl, 1-, 2- or 3-piperazinyl, 1,2,3,4-tetrahydro-1-, -2-, -3-, -4-, -5-, -6-, -7- or -8-quinolyl or 1,2,3,4-tetrahydro-1-, -2-, -3-, -4-, -5-, -6-, -7- or -8-isoquinolyl, each of which is monosubstituted or disubstituted by COOH, CH₂COOH, COOCH₃, COOC₂H₅, CONH₂, CON(CH₃)₂, CONHCH₃ or CN,
A, A' are each, independently of one another, H or alkyl having from 1 to 6 carbon atoms,
Hal is F, Cl, Br or I,
and
n is 0, 1, 2 or 3,
and physiologically acceptable salts thereof.

2. Compounds of the formula I according to Claim 1
(a) 4-[4-(3,4-methylenedioxybenzylamino)-5,6,7,8-tetrahydro[1]-benzothieno[2,3-d]pyrimidin-2-yl]benzoic acid;
(b) 4-[4-(3,4-methylenedioxybenzylamino)-6-methylthieno[2,3-d]-pyrimidin-2-yl]benzoic acid;
(c) 4-[4-(3,4-methylenedioxybenzylamino)-5,6-dimethylthieno-[2,3-d]pyrimidin-2-yl]benzoic acid;
(d) 4-[4-(3,4-methylenedioxybenzylamino)-6-chlorothieno[2,3-d]-pyrimidin-2-yl]benzoic acid;
(e) 4-[4-(3-chloro-4-methoxybenzylamino)-5,6,7,8-tetrahydro[1]-benzothieno[2,3-d]pyrimidin-2-yl]benzoic acid;
(f) 1-[4-(3,4-methylenedioxybenzylamino)-5,6,7,8-tetrahydro[1]-benzothieno[2,3-d]pyrimidin-2-yl]piperidine-4-carboxylic acid;
(g) 1-[4-(3,4-methylenedioxybenzylamino)-6-methylthieno[2,3-d]-pyrimidin-2-yl]piperidine-4-carboxylic acid;
(h) 4-[[4-(3,4-methylenedioxybenzylamino)-5,6,7,8-tetrahydro[1]-benzothieno[2,3-d]pyrimidin-2-yl]cyclohexanecarboxylic acid;
and physiologically acceptable salts thereof.

3. Process for the preparation of compounds of the formula I according to Claim 1 and salts thereof, **characterised in that** an acidic compound of the formula I is converted into one of its salts by treatment with a base.

4. Process for the preparation of pharmaceutical preparations, **characterised in that** a compound of the formula I according to Claim 1 and/or one of its physiologically acceptable salts is converted into a suitable dosage form together with at least one solid, liquid or semiliquid excipient or adjuvant.

5. Pharmaceutical preparation, **characterised by** a content of at least one compound of the formula I according to Claim 1 and/or one of its physiologically acceptable salts.

6. Compounds of the formula I according to Claim 1 and physiologically acceptable salts thereof for combating diseases of the cardiovascular system and for the treatment and/or therapy of potency disorders.

7. Medicaments of the formula I according to Claim 1 and physiologically acceptable salts thereof as phosphodiesterase V inhibitors.

8. Use of compounds of the formula I according to Claim 1 and/or physiologically acceptable salts thereof for the preparation of a medicament.

9. Use of compounds of the formula I according to Claim 1 and/or physiologically acceptable salts thereof for the preparation of a medicament for combating diseases.

10. Use of compounds of the formula I according to Claim 1 and/or physiologically acceptable salts thereof for the preparation of a medicament for the treatment of diseases of the cardiovascular system, in particular cardiac insufficiency, and for the treatment and/or therapy of potency disorders (erectile dysfunction).

## Revendications

1. Composés de formule I 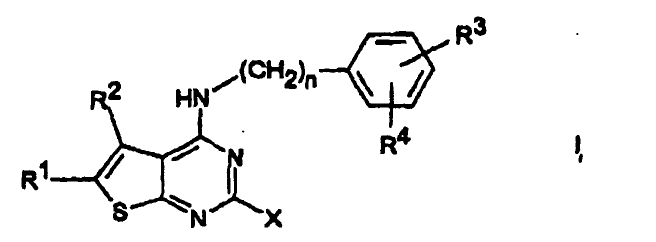 dans laquelle
R¹, R² sont chacun, indépendamment l'un de l'autre, H, A, OA, alkényle, alkynyle, CF₃ ou Hal,
dans lequel l'un parmi les radicaux R¹ et R² est toujours ≠ H,
R¹ et R² ensemble sont alternativement alkylène ayant de 3 à 5 atomes de carbone,
R³, R⁴ sont chacun, indépendamment l'un de l'autre, H, A, OA, NO₂, NH₂, NHA, NAA' ou Hal,
R³ et R⁴ ensemble sont alternativement -O-CH₂-CH₂-, -O-CH₂-O- ou -O-CH₂-CH₂-O-,
X is phényle, cyclopentyle, cyclohexyle, cycloheptyle, 2,3-dihydro-2-, -3-, -4- ou -5-furyle, 2,5-dihydro-2-, -3-, -4- ou -5-furyle, tétrahydro-2- ou -3-furyle, 1,3-dioxolan-4-yle, tétrahydro-2- ou -3-thiényle, 2,3-dihydro-1-, -2-, -3-, -4- ou -5-pyrrolyle, 2,5-dihydro-1-, -2-, -3-, -4- ou -5-pyrrolyle, 1-, 2- ou 3-pyrrolidinyle, tétrahydro-1-, -2- ou -4-imidazolyle, 2,3-dihydro-1-, -2-, -3-, -4- ou -5-pyrazolyle, tétrahydro-1-, -3- ou -4-pyrazolyle, 1,4-dihydro-1-, -2-, -3- ou -4-pyridyle, 1,2,3,4-tétrahydro-1-, -2-, -3-, -4-, -5- ou -6-pyridyle, 1-, 2-, 3- ou 4-pipéridinyle, 2-, 3- ou 4-morpholinyle, tétrahydro-2-, -3- ou -4-pyranyle, 1,4-dioxanyle, 1,3-dioxan-2-, -4- ou -5-yle, hexahydro-1-, -3- ou -4-pyridazinyle, hexahydro-1-, -2-, -4- ou -5-pyrimidinyle, 1-, 2- ou 3-pipérazinyle, 1,2,3,4-tétrahydro-1-, -2-, -3-, -4-, -5-, -6-, -7- ou -8-quinoléinyle ou 1,2,3,4-tétrahydro-1-, -2-, -3-, -4-, -5-, -6-, -7- ou -8-isoquinoléinyle, chacun d'entre eux étant mono-substitué ou disubstitué par COOH, CH₂COOH, COOCH₃, COOC₂H₅, CONH₂, CON(CH₃)₂, CONHCH₃ ou CN,
A, A' sont chacun, indépendamment l'un de l'autre, H ou alkyle ayant 1 à 6 atomes de carbone,
Hal est F, Cl, Brou I,
et
n vaut 0, 1, 2 ou 3,
et les sels physiologiquement acceptables de ceux-ci.

2. Composés de formule I selon la revendication 1
(a) l'acide 4-[4-(3,4-méthylènedioxybenzylamino)-5,6,7,8-tétrahydro[1]benzothiéno[2,3-d]pyrimidin-2-yl]benzoïque ;
(b) l'acide 4-[4-(3,4-méthylènedioxybenzylamino)-6-méthylthiéno-[2,3-d]pyrimidin-2-yl]benzoïque ;
(c) l'acide 4-[4-(3,4-méthylènedioxybenzylamino)-5,6-diméthylthiéno[2,3-d]pyrimidin-2-yl]benzoïque ;
(d) l'acide 4-[4-(3,4-méthylènedioxybenzylamino)-6-chlorothiéno-[2,3-d]pyrimidin-2-yl]benzoïque ;
(e) l'acide 4-[4-(3-chloro-4-méthoxybenzylamino)-5,6,7,8-tétrahydro[1]benzothiéno[2,3-d]pyrimidin-2-yl]benzoïque ;
(f) l'acide 1-[4-(3,4-méthylènedioxybenzylamino)-5,6,7,8-tétrahydro[1]benzothiéno[2,3-d]pyrimidin-2-yl]pipéridine-4-carboxylique ;
(g) l'acide 1-[4-(3,4-méthylènedioxybenzylamino)-6-méthylthiéno-[2,3-d]pyrimidin-2-yl]pipéridine-4-carboxylique ;
(h) l'acide 4-[[4-(3,4-méthylènedioxybenzylamino)-5,6,7,8-tétrahydro[1]benzothiéno[2,3-d]pyrimidin-2-yl]cyclohexanecarboxylique ;
et les sels physiologiquement acceptables de ceux-ci.

3. Procédé de préparation de composés de formule I selon la revendication 1 et de sels de ceux-ci, **caractérisé en ce qu'**un composé acide de formule I est converti en l'un de ses sels par un traitement par une base.

4. Procédé de préparation de préparations pharmaceutiques, **caractérisé en ce qu'**un composé de formule I selon la revendication 1 et/ou l'un de ses sels physiologiquement acceptables est mis sous une forme de dosage convenable conjointement avec au moins un excipient ou adjuvant solide, liquide ou semi-liquide.

5. Préparation pharmaceutique, **caractérisée par** une teneur en au moins un composé de formule I selon la revendication 1 et/ou en l'un de ses sels physiologiquement acceptables.

6. Composés de formule I selon la revendication 1 et les sels physiologiquement acceptables de ceux-ci pour lutter contre les maladies du système cardiovasculaire et pour le traitement et/ou la thérapie des troubles de la virilité.

7. Médicaments de formule I selon la revendication 1 et les sels physiologiquement acceptables de ceux-ci comme inhibiteurs de la phosphodiestérase V.

8. Utilisation de composés de formule I selon la revendication 1 et/ou de sels physiologiquement acceptables de ceux-ci pour la préparation d'un médicament.

9. Utilisation de composés de formule I selon la revendication 1 et/ou de sels physiologiquement acceptables de ceux-ci pour la préparation d'un médicament pour lutter contre des maladies.

10. Utilisation de composés de formule I selon la revendication 1 et/ou de sels physiologiquement acceptables de ceux-ci pour la préparation d'un médicament pour le traitement de maladies du système cardiovasculaire, en particulier l'insuffisance cardiaque, et pour le traitement et/ou la thérapie des troubles de la virilité (dysfonctionnement érectile).
